# EUROPEAN PATENT APPLICATION

(11) **EP 4 728 968 A1**
(43) Date of publication of application: **22.04.2026**
(21) Application number: 24839936.2
(22) Date of filing: 21.06.2024
(51) Int. Cl.: A61B 5/00, G06T 7/11, G16H 30/40

(54) **ORAL IMAGE PROCESSING DEVICE AND OPERATING METHOD OF ORAL IMAGE PROCESSING DEVICE**

(30) Priority: 13.07.2023 KR 20230091352; 20.06.2024 KR 20240080581
(71) Applicant: Medit Corp., Seoul 07207 (KR)
(72) Inventor: BAEK, Jongwoong, Seoul 07207 (KR); KO, Kinam, Seoul 07207 (KR)
(74) Representative: Kim Kang, Jae Hee
(86) International application number: PCT/KR2024/008641
(87) International publication number: WO 2025/014122

(57) **Abstract**

This operating method of an oral image processing device may comprise the steps of: acquiring an oral image including a plurality of oral elements; acquiring a segmentation map in which each of a plurality of oral element areas corresponding to each of a plurality of oral elements is segmented on the basis of the oral image; identifying, from the segmentation map, a caries area corresponding to dental caries from among the plurality of oral element areas; dilating the caries area so as to acquire a correction segmentation map in which the caries area from among the plurality of oral element areas is expanded; and providing, on the basis of the correction segmentation map, a result image in which the dental caries from among the plurality of oral elements is expanded and displayed more than the identified caries area.

## Description

### Technical Field

The disclosed embodiments relate to an oral image processing device and a method of operating the oral image processing device, and more particularly, to an oral image processing device for providing a result image in which a plurality of oral elements included in an oral image are distinguished from each other, and a method of operating the oral image processing device.

### Background Art

In dental treatment, particularly in treatments such as prosthetics, dental computer aided design/computer aided manufacturing (CAD/CAM) has been widely used. In dental treatment using CAD/CAM, the most important factor is to obtain exact two-dimensional or three-dimensional data regarding the shapes of target objects such as a patient's teeth, gums, dental caries, or the like.

For example, one of methods of the related art for obtaining two-dimensional or three-dimensional data regarding the shape of dental caries uses an oral scanner that employs a fluorescence response at a specific wavelength or a near-infrared light source. In this case, hardware modifications are required for the oral scanner and a scan tip.

To acquire raw data through the method, an infrared-light source or a separate device emitting a specific wavelength is required other than a general color camera, which may cause inconvenience to patients and incur additional costs.

In addition, when acquiring two-dimensional or three-dimensional data including target objects such as dental caries, based on acquired raw data, users may fail to detect small target objects or target objects obscured by other objects.

### Disclosure of Invention

### Technical Problem

Embodiments aim to provide a method of operating an oral image processing device for obtaining a result image, in which oral elements including dental caries are distinguished, by using an oral image captured with a general color camera used to scan an oral cavity, and to provide a device for performing operations according to the method.

In addition, embodiments aim to provide a method of operating an oral image processing device for displaying dental caries distinguished in a result image by dilating the dental caries or displaying the dental caries in a color different from that of an oral image, and to provide a device for performing operations according the method.

### Solution to Problem

To solve the technical problem, an embodiment of the present disclosure provides a method of operating an oral image processing device. The method of operating an oral image processing device may include acquiring an oral image including a plurality of oral elements. The method of operating an oral image processing device may include acquiring, based on the oral image, a segmentation map in which a plurality of oral element regions respectively corresponding to the plurality of oral elements are defined by segmentation. The method of operating an oral image processing device may include identifying a caries region corresponding to dental caries among the plurality of oral element regions in the segmentation map. The method of operating an oral image processing device may include dilating the caries region to acquire an adjusted segmentation map in which the caries region among the plurality of oral element regions is dilated. The method of operating an oral image processing device may include providing, based on the adjusted segmentation map, a result image in which the dental caries among the plurality of oral elements is dilated compared with the identified caries region.

In an embodiment of the present disclosure, the providing of the result image may include generating, based on the oral image and the adjusted segmentation map, a three-dimensional oral image in which the dental caries among the plurality of oral elements is dilated compared with the identified caries region. The providing of the result image may include providing the three-dimensional oral image as the result image.

In an embodiment of the present disclosure, the method of operating an oral image processing device may include extracting, from the segmentation map, a caries masking map including information on the caries region corresponding to the dental caries among the plurality of oral elements. The method of operating an oral image processing device may include generating an adjusted caries masking map by dilating the caries region included in the caries masking map. The acquiring of the adjusted segmentation map may include acquiring, based on the adjusted caries masking map and the segmentation map, the adjusted segmentation map in which the caries region is dilated.

According to an embodiment of the present disclosure, in the method of operating an oral image processing device, among the plurality of oral regions included in the adjusted segmentation map, the dilated caries region may have a first weight, and remaining regions may have a second weight The first weight may be greater than the second weight.

In an embodiment of the present disclosure, the oral image may include a plurality of oral images. The adjusted segmentation map may include a plurality of adjusted segmentation maps respectively corresponding to the plurality of oral images The dilated caries region in each of the plurality of adjusted segmentation maps may have the first weight. The remaining regions in each of the plurality of adjusted segmentation maps may have the second weight. In the generating of the three-dimensional oral image, all of the plurality of adjusted segmentation maps may be integrated to generate the three-dimensional oral image such that a region, among the plurality of oral regions, having the first weight greater than the second weight may be displayed as the dilated caries region.

In an embodiment of the present disclosure, the generating of the three-dimensional oral image may include generating a plurality of point clouds based on the plurality of oral images. The generating of the three-dimensional oral image may include generating, based on the plurality of point clouds and the plurality of adjusted segmentation maps, the three-dimensional oral image including a plurality of voxels including the plurality of point clouds.

In an embodiment of the present disclosure, the method of operating an oral image processing device may include generating a plurality of meshes including information on the three-dimensional oral image by using the plurality of voxels. The method of operating an oral image processing device may include generating a post-processed three-dimensional oral image by using the plurality of meshes.

In an embodiment of the present disclosure, the acquiring of the segmentation map may include providing the oral image as an input to a segmentation map extraction module and acquiring the segmentation map as an inference result of the segmentation map extraction module The segmentation map extraction module may include an artificial intelligence model pretrained using data sets, each of the data sets including a training oral image including a plurality of oral elements and labeling information in which the plurality of oral elements included in the training oral images may be labeled.

According to an embodiment of the present disclosure, in the acquiring of the segmentation map, tooth regions corresponding to teeth and the caries region corresponding to the dental caries may be defined by selective segmentation based on the acquired oral image to acquire the segmentation map.

In an embodiment of the present disclosure, the providing of the result image may include acquiring a selection input for selecting at least one oral element among the plurality of oral elements. The providing of the result image may include providing, based on the selection input, the result image such that a region corresponding to the selected at least one oral element may be displayed in a second color different from a first color representing the oral image.

To solve the technical problem, an embodiment of the present disclosure provides an oral image processing device. The oral image processing device may include a memory storing at least one instruction. The oral image processing device may include at least one processor executing the at least one instruction stored in the memory. The at least one processor may execute the at least one instruction to acquire an oral image including a plurality of oral elements. The at least one processor may execute the at least one instruction to acquire, based on the oral image, a segmentation map in which a plurality of oral element regions respectively corresponding to the plurality of oral elements are defined by segmentation. The at least one processor may execute the at least one instruction to identify a caries region corresponding to dental caries among the plurality of oral element regions in the segmentation map. The at least one processor may execute the at least one instruction to dilate the caries region to acquire an adjusted segmentation map in which the caries region among the plurality of oral element regions is dilated . The at least one processor may execute the at least one instruction to provide, based on the adjusted segmentation map, a result image in which the dental caries among the plurality of oral elements is dilated compared with the identified caries region.

In an embodiment of the present disclosure, the at least one processor may execute the at least one instruction to generate, based on the oral image and the adjusted segmentation map, a three-dimensional oral image in which the dental caries among the plurality of oral elements is dilated compared with the identified caries region The at least one processor may execute the at least one instruction to provide the three-dimensional oral image as the result image.

In an embodiment of the present disclosure, the at least one processor may execute the at least one instruction to extract, from the segmentation map, a caries masking map including information on the caries region corresponding to the dental caries among the plurality of oral elements. The at least one processor may execute the at least one instruction to generate an adjusted caries masking map by dilating the caries region included in the caries masking map. The at least one processor may execute the at least one instruction to acquire, based on the adjusted caries masking map and the segmentation map, the adjusted segmentation map in which the caries region is dilated.

In an embodiment of the present disclosure, the at least one processor may execute the at least one instruction such that among the plurality of oral regions included in the adjusted segmentation map, the dilated caries region may have a first weight, and remaining regions may have a second weigh. The first weight may be greater than the second weight.

In an embodiment of the present disclosure, the oral image may include a plurality of oral images. The adjusted segmentation map may include a plurality of adjusted segmentation maps respectively corresponding to the plurality of oral images. In each of the plurality of adjusted segmentation maps, the dilated caries region may have the first weight, and the remaining regions may have the second weight. The at least one processor may execute the at least one instruction to generate the three-dimensional oral image by integrating all of the plurality of adjusted segmentation maps such that a region, among the plurality of oral regions, having the first weight greater than the second weight may be displayed as the dilated caries region.

In an embodiment of the present disclosure, the at least one processor may execute the at least one instruction to generate a plurality of point clouds based on the plurality of oral images. The at least one processor may execute the at least one instruction to generate, based on the plurality of point clouds and the plurality of adjusted segmentation maps, the three-dimensional oral image including a plurality of voxels including the plurality of point clouds.

In an embodiment of the present disclosure, the at least one processor may execute the at least one instruction to generate a plurality of meshes including information on the three-dimensional oral image by using the plurality of voxels The at least one processor may execute the at least one instruction to generate a post-processed three-dimensional oral image by using the plurality of meshes.

In an embodiment of the present disclosure, the at least one processor may execute the at least one instruction to provide the oral image as an input to a segmentation map extraction module and acquire the segmentation map as an inference result of the segmentation map extraction module. The segmentation map extraction module may include an artificial intelligence model pretrained using data sets, each of the data sets including a training oral image including a plurality of oral elements and labeling information in which the plurality of oral elements included in the training oral images may be labeled.

In an embodiment of the present disclosure, the at least one processor may execute the at least one instruction to acquire a selection input for selecting at least one oral element among the plurality of oral elements. The at least one processor may execute the at least one instruction to provide, based on the selection input, the result image such that a region corresponding to the selected at least one oral element may be displayed in a second color different from a first color representing the oral image.

An embodiment of the present disclosure may provide a non-transitory computer-readable recording medium storing at least one instruction executable by at least one processor of an oral image processing device, wherein the at least one processor of the oral image processing device executes the at least one instruction to cause the oral image processing device to acquire an oral image including a plurality of oral elements, acquire, based on the oral image, a segmentation map in which a plurality of oral element regions respectively corresponding to the plurality of oral elements are defined by segmentation, identify a caries region corresponding to dental caries among the plurality of oral element regions in the segmentation map, dilate the caries region to acquire an adjusted segmentation map in which the caries region among the plurality of oral element regions is dilated, and provide, based on the adjusted segmentation map, a result image in which the dental caries among the plurality of oral elements may be displayed as the dilated caries region larger than the identified caries region.

### Advantageous Effects of Invention

In an oral image processing device and a method of operating the oral image processing device according to embodiments, a result image in which oral elements including dental caries are distinguished from each other may be obtained based on an oral image acquired by scanning an oral cavity. Accordingly, the result image in which dental caries is distinguished may be obtained without separate equipment.

In addition, in the oral image processing device and the method of operating the oral image processing device according to the embodiments, dental caries distinguished in the result image may be displayed either by dilating the dental caries or by displaying the dental caries in a color different from that of an oral image, thereby enhancing user visibility of the dental caries included in the result image.

### Brief Description of Drawings

The present disclosure may be readily understood by the following detailed description in conjunction with the accompanying drawings, in which reference numerals refer to structural elements.
FIG. 1A is a view illustrating an oral image processing device according to an embodiment of the present disclosure.
FIG. 1B is a view illustrating an oral image processing device according to an embodiment of the present disclosure.
FIG. 2 is a block diagram illustrating a configuration of an oral image processing device according to an embodiment of the present disclosure.
FIG. 3A is a flowchart illustrating a method of operating an oral image processing device, according to an embodiment of the present disclosure.
FIG. 3B is a flowchart illustrating a method of operating an oral image processing device, according to an embodiment of the present disclosure.
FIG. 4 is a view illustrating an operation of acquiring a segmentation map from an oral image, according to an embodiment of the present disclosure.
FIG. 5 is a view illustrating an operation of extracting a caries masking map including information on a caries region corresponding to dental caries, according to an embodiment of the present disclosure.
FIG. 6 is a view illustrating an operation of dilating a caries region, according to an embodiment of the present disclosure.
FIG. 7 is a view illustrating an operation of acquiring an adjusted segmentation map in which a caries region is dilated, according to an embodiment of the present disclosure.
FIG. 8 is a view illustrating a segmentation map, a caries masking map, an adjusted caries masking map, and an adjusted segmentation map, according to an embodiment of the present disclosure.
FIG. 9 is a flowchart illustrating an operation of generating a three-dimensional oral image based on an oral image and an adjusted segmentation map, according to an embodiment of the present disclosure.
FIG. 10 is a view illustrating an operation of generating a three-dimensional oral image based on a plurality of adjusted segmentation maps and a plurality of oral images, according to an embodiment of the present disclosure.
FIG. 11 is a view illustrating an operation of providing a three-dimensional oral image, according to an embodiment of the present disclosure.
FIG. 12 is a view illustrating an operation of providing a three-dimensional oral image, according to an embodiment of the present disclosure.
FIG. 13 is a flowchart illustrating an operation of displaying at least one oral element in a color different from that of an oral image, according to an embodiment of the present disclosure.
FIG. 14 is a view illustrating an operation of displaying at least one oral element in a first color or a second color, according to an embodiment of the present disclosure.
FIG. 15 is a view illustrating an operation of an oral image processing system according to an embodiment of the present disclosure.

### Mode for the Invention

The present specification describes the principles of the present disclosure and discloses embodiments so as to clearly define the scope of the present disclosure and enable those of ordinary skill in the art to which the present disclosure pertains to practice the present disclosure. The disclosed embodiments may be implemented in various forms.

Throughout the specification, like reference numerals refer to like elements. The present specification does not describe all elements of the embodiments, and general content in the art to which the present disclosure pertains or content duplicated among the embodiments is omitted. As used in the specification, "parts" or "portions" may be implemented as software or hardware, and in some embodiments, a plurality of "parts" may be implemented as one unit or element, or one "part" may include a plurality of elements. Hereinafter, operational principles and embodiments of the present disclosure will be described with reference to the accompanying drawings.

In the present specification, the term "image" may refer to an image (hereinafter referred to as an "oral image") showing at least one tooth or an oral cavity including at least one tooth.

Furthermore, in the present specification, the term "image" may refer to a two-dimensional image of a target object, or a three-dimensional model or image representing the target object in three dimensions. Furthermore, in the present specification, the term "image" may refer to data required to represent a target object two-dimensionally or three-dimensionally, for example, raw data acquired using at least one image sensor. Specifically, raw data may refer to data acquired to generate an image, and when a target object is scanned using a three-dimensional scanner, data (for example, two-dimensional data) acquired using at least one image sensor included in the three-dimensional scanner may be raw data.

In the present specification, the term "oral element" may include a tooth, a gum, at least a region of the oral cavity, and/or an artificial structure insertable into the oral cavity (for example, an orthodontic appliance, an implant, an artificial tooth, an orthodontic auxiliary tool inserted into the oral cavity, or the like). Here, the orthodontic appliance may include at least one of a bracket, an attachment, an orthodontic screw, a lingual orthodontic appliance, and a removable orthodontic retainer.

In the present specification, a "result image" may include various polygon meshes. For example, when two-dimensional data is acquired using an oral scanner, an oral image processing device may calculate coordinates of a plurality of illuminated surface points by using a triangulation method. As the amount of scan data increases by scanning the surface of an oral element while moving an oral scanner, coordinates of surface points may be accumulated. As a result of such image acquisition, a point cloud of vertices may be identified to represent the extent of a surface. Points in a point cloud may be actually measured points on a three-dimensional surface of an object. A surface structure may be approximated by forming polygon meshes in which adjacent vertices of a point cloud are connected by line segments. The polygon meshes may be determined in various forms, such as triangular meshes, quadrilateral meshes, pentagonal meshes, or the like. Polygons of such a mesh model and relationships between neighboring polygons may be used to extract tooth-boundary features, such as curvature, minimum curvature, edges, spatial relationships, or the like.

Hereinafter, embodiments will be described in detail with reference to the accompanying drawings.

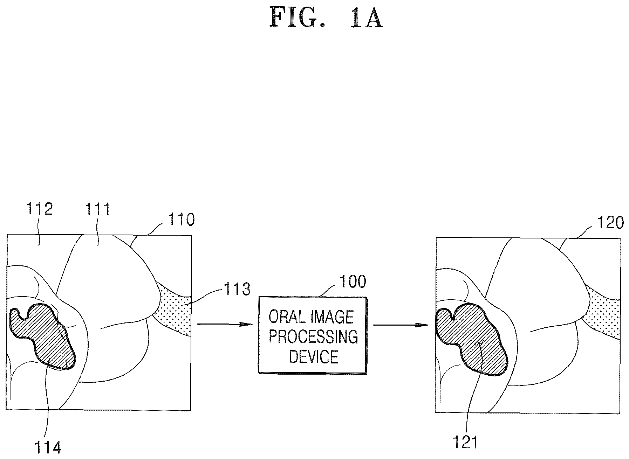

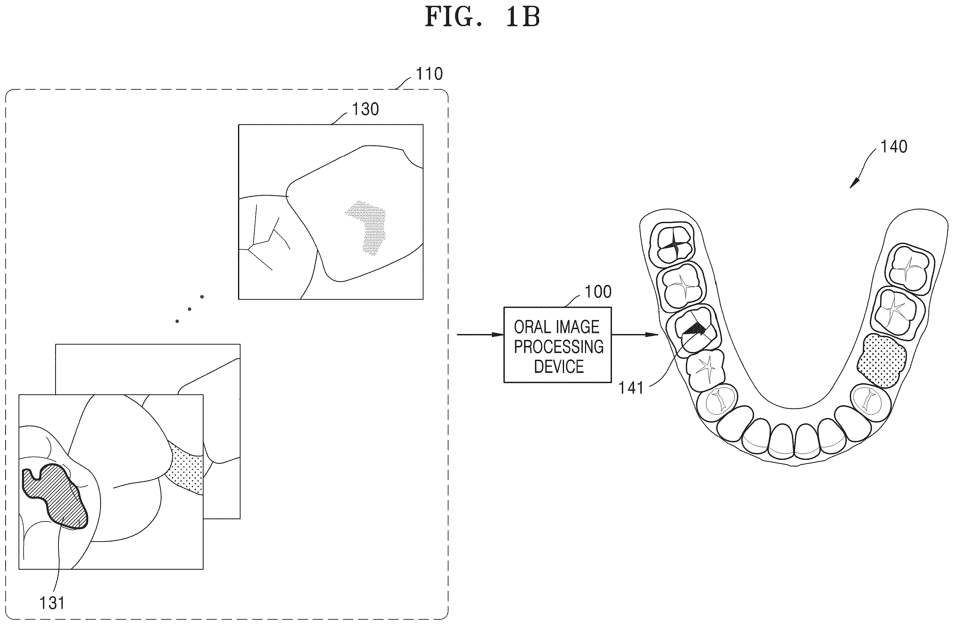

FIG. 1A is a view illustrating an oral image processing device according to an embodiment of the present disclosure. FIG. 1B is a view illustrating an oral image processing device according to an embodiment of the present disclosure.

Referring to FIG. 1A, an oral image processing device 100, an oral image 110, and a result image 120 are illustrated according to an embodiment of the present disclosure.

In an embodiment of the present disclosure, the oral image 110 may include a plurality of oral elements. In an embodiment of the present disclosure, the plurality of oral elements may include at least one of teeth, gums, dental caries, artificial structures (for example, orthodontic appliances including brackets and wires, implants, artificial teeth, orthodontic auxiliary tools inserted into the oral cavity, or the like), and prostheses (for example, inlays, crowns, bridges, or the like).

In an embodiment of the present disclosure, the oral image 110 may be an image acquired using an electronic device (for example, an oral scanner) configured to scan the oral cavity.

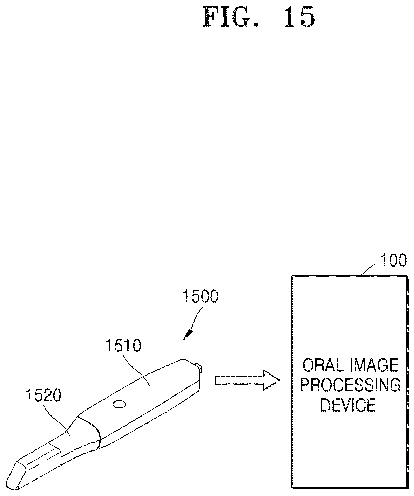

In an embodiment of the present disclosure, the oral image processing device 100 may acquire the oral image 110 by using an oral scanner connected via a communication interface. In an embodiment of the present disclosure, the oral image processing device 100 may acquire the oral image 110 by using an oral scanner connected via a wired or wireless interface. However, the present disclosure is not limited thereto, and the oral image processing device 100 may acquire the oral image 110 from an external server connected through a communication interface. Acquisition of the oral image 110 will be described later with reference to FIG. 15.

In an embodiment of the present disclosure, the oral image processing device 100 may be implemented as an electronic device in various forms, such as a smartphone, a laptop computer, a desktop computer, a notebook, a PDA, a tablet PC, or the like. The oral image processing device 100 may also be implemented as an electronic device in the form of a server (or server device) for processing oral images.

In an embodiment of the present disclosure, the oral image processing device 100 may generate, based on the acquired oral image 110, a result image 120 in which a region corresponding to a specific oral element among the plurality of oral elements is dilated. Based on the acquired oral image 110, the oral image processing device 100 may provide, to a user of the oral image processing device 100, the result image 120 in which a caries region corresponding to dental caries among the plurality of oral elements is dilated.

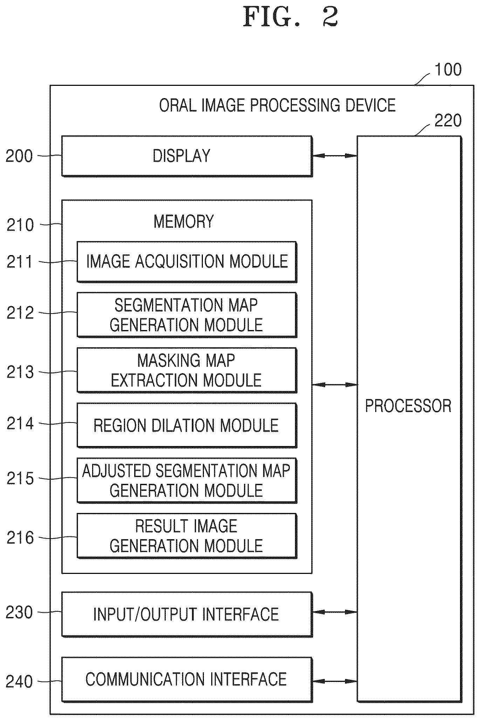

In an embodiment of the present disclosure, the oral image 110 may be a two-dimensional image. Based on the oral image 110, which is a two-dimensional image, the oral image processing device 100 may provide a two-dimensional result image 120 in which dental caries 114 among the plurality of oral elements is dilated. However, the present disclosure is not limited thereto. Based on the oral image 110, which is a two-dimensional image, the oral image processing device 100 may also provide a three-dimensional oral image 140 (refer to FIG. 1B) in which dental caries 114 is dilated among the plurality of oral elements. Hereinafter, operations of the oral image processing device 100 for generating result images 120 and 140 will be described with reference to FIGS. 2, 3, and 7 to 9.

In an embodiment of the present disclosure, the oral image 110 illustrated in FIG. 1A includes teeth 111, a gum 112, a prosthesis 113, and the dental caries 114. Based on the acquired oral image 110, the oral image processing device 100 may provide the result image 120 in which the teeth 111, the gum 112, the prosthesis 113, and the dental caries 114 included in the oral image 110 are distinguished from each other.

Although FIG. 1A illustrates the result image 120 in which a caries region 121 is dilated to emphasize the dental caries 114, the present disclosure is not limited thereto. A result image 120 in which another distinguished oral element is emphasized in addition to the dilated caries region 121 may also be provided by changing setting values of the oral image processing device 100. In this case, the oral image processing device 100 may emphasize an oral element with a color different from colors included in the oral image 110 (for example, a color that is easily noticeable to users).

In an embodiment of the present disclosure, the oral image processing device 100 may segment the oral image 110 into a plurality of oral element regions respectively corresponding to the plurality of oral elements included in the oral image 110. The oral image processing device 100 may dilate a specific region (for example, a caries region corresponding to dental caries) corresponding to a specific oral element among the plurality of oral element regions. The oral image processing device 100 may generate and provide the result image 120 in which the dilated specific region is reflected.

In an embodiment of the present disclosure, the oral image processing device 100 may increase visibility of the specific oral element among the plurality of oral elements included in the oral image 110 by providing the result image 120 including the dilated specific region. The oral image processing device 100 may emphasize a specific oral element among the plurality of oral elements included in the oral image 110.

Referring to FIG. 1B, in an embodiment of the present disclosure, an oral image 110 may include a plurality of oral images 130 acquired by scanning the oral cavity while changing the position of an oral scanner within the oral cavity.

In an embodiment of the present disclosure, the oral image processing device 100 may provide, based on the plurality of oral images 130, a three-dimensional oral image 140 in which a region corresponding to a specific oral element among a plurality of oral elements is dilated.

In an embodiment of the present disclosure, the three-dimensional oral image 140 may be generated by three-dimensionally modeling the oral cavity based on the plurality of oral images 130. The oral image processing device 100 may provide the three-dimensional oral image 140 by using three-dimensional data (for example, surface data, mesh data, or the like) that is generated based on the plurality of oral images 130 to represent the shape of the oral cavity three-dimensionally.

In an embodiment of the present disclosure, the oral image processing device 100 may dilate a region (for example, a caries region) corresponding to a specific oral element (for example, dental caries 131) included in the plurality of oral images 130. The oral image processing device 100 may provide the three-dimensional oral image 140 in which a dilated region (for example, a dilated caries region 141) corresponding to a specific oral element is reflected. In this case, the dilated caries region 141 corresponding to the specific oral element may be displayed in a color different from colors included in the plurality of oral images 130, thereby enhancing visibility of the specific oral element.

FIG. 2 is a block diagram illustrating a configuration of the oral image processing device 100 according to an embodiment of the present disclosure.

Referring to FIGS. 1A, 1B, and 2, in an embodiment of the present disclosure, the oral image processing device 100 may include a display 200, memory 210, at least one processor 220, an input/output interface 230, and a communication interface 240. However, not all of the components illustrated in FIG. 2 are essential components.

In an embodiment of the present disclosure, the oral image processing device 100 may be implemented with more or fewer components than illustrated in FIG. 2. The display 200, the memory 210, the at least one processor 220, the input/output interface 230, and the communication interface 240 may be electrically and/or physically connected to each other.

In an embodiment of the present disclosure, the display 200 may display the result images 120 and 140 under control by the at least one processor 220. Specifically, the display 200 may display an interface screen including the result images 120 and 140 generated by the oral image processing device 100. However, the present disclosure is not limited thereto, and the display 200 may also display an interface screen including information related to dental treatment for a patient. In this case, a user of the oral image processing device 100 may refer to a person (for example, a doctor) who scans the oral cavity of a patient using an electronic device such as an oral scanner.

In an embodiment of the present disclosure, the memory 210 may store instructions, data structures, and program code readable by the at least one processor 220. Instructions, algorithms, data structures, program code, and application programs stored in the memory 210 may be implemented using programming or scripting languages such as C, C++, Java, assembler, or the like.

In an embodiment of the present disclosure, the memory 210 may be implemented as at least one unit. In disclosed embodiments, operations of the oral image processing device 100 may be implemented by the at least one processor 220 executing instructions or code of programs stored in the memory 210.

In an embodiment of the present disclosure, the memory 210 may not be separately provided but may be included as internal memory in the at least one processor 220.

In an embodiment of the present disclosure, the memory 210 may store various types of modules that may be used to perform operations of the oral image processing device 100 shown in FIGS. 1A to 16.

In an embodiment of the present disclosure, the memory 210 may store various types of modules that may be used to provide a result image 120 or 140 in which a plurality of oral elements included in an oral image 110 are distinguished from each other by the oral image processing device 100. The memory 210 may store various types of modules that may be used to generate a result image 120 or 140 in which a specific region corresponding to a specific oral element is dilated among a plurality of oral element regions defined by segmentation.

In an embodiment of the present disclosure, the memory 210 may store an image acquisition module 211, a segmentation map generation module 212, a masking map extraction module 213, a region dilation module 214, an adjusted segmentation-map generation module 215, and a result image generation module 216. However, not all modules illustrated in FIG. 2 are essential modules. The memory 210 may store more or fewer modules than illustrated in FIG. 2.

In an embodiment of the present disclosure, the "modules" included in the memory 210 may each refer to a unit configured to process a function or operation that the at least one processor 220 performs. The "modules" included in the memory 210 may be implemented as software such as instructions, algorithms, data structures, or program code.

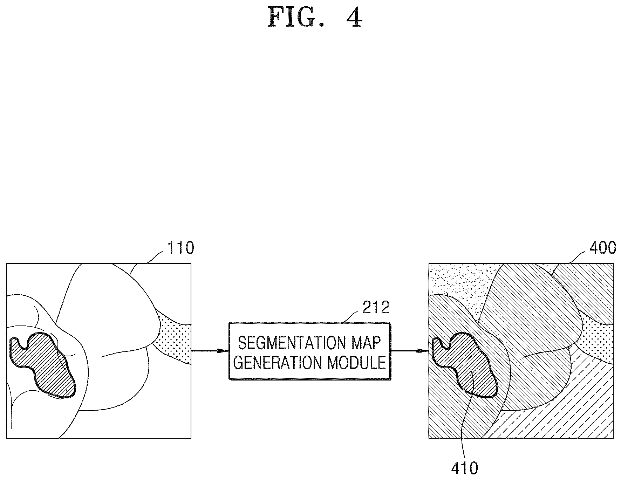

Hereinafter, the image acquisition module 211, the segmentation map generation module 212, the masking map extraction module 213, the region dilation module 214, the adjusted segmentation map generation module 215, and the result image generation module 216 included in the memory 210 will be described later with reference to FIGS. 4 to 7 and FIG. 9.

In an embodiment of the present disclosure, the at least one processor 220 may be a component including one or more processors and configured to control a series of processes for the oral image processing device 100 to operate according to embodiments described below.

In an embodiment of the present disclosure, the at least one processor 220 may include at least one of central processing units, microprocessors, graphics processing units, application processors (APs), application specific integrated circuits (ASICs), digital signal processors (DSPs), digital signal processing devices (DSPDs), programmable logic devices (PLDs), field-programmable gate arrays (FPGAs), communication processors (CPs), neural processing units, and artificial intelligence-dedicated processors each designed with a hardware structure specialized for training and inference of an artificial intelligence (AI) model. However, the at least one processor 220 is not limited thereto.

In an embodiment of the present disclosure, when one or more processors included in the at least one processor 220 are artificial intelligence-dedicated processors, the artificial intelligence-dedicated processors may be designed with a hardware structure specialized for processing a specific artificial intelligence model.

In an embodiment of the present disclosure, the at least one processor 220 may include circuitry such as a system-on-chip (SoC) or an integrated circuit (IC).

In an embodiment of the present disclosure, the at least one processor 220 may execute various types of modules stored in the memory 210. The at least one processor 220 may execute at least one instruction constituting various types of modules stored in the memory 210. The at least one processor 220 may control operations of the oral image processing device 100 by executing programs or at least one instruction stored in the memory 210.

In an embodiment of the present disclosure, the input/output interface 230 may be connected to an external input device (for example, a keyboard, a mouse, a touch pad, a microphone, or the like) or an external electronic device (for example, an oral scanner or the like). The at least one processor 220 may acquire a user input that is provided to the oral image processing device 100 from an external input device through the input/output interface 230.

The at least one processor 220 may acquire an oral image 110 from an external electronic device through the input/output interface 230. The at least one processor 220 may also provide a generated result image 120 or 140 to an external electronic device through the communication interface 240.

In an embodiment of the present disclosure, the input/output interface 230 may perform input/output operations with an external input device or an external electronic device by using at least one input/output interface type including a high-definition multimedia interface (HDMI) port, a digital visual interface (DVI), a component jack, a PC port, or a universal serial bus (USB) port. However, the present disclosure is not limited to the input/output interface types described above.

In an embodiment of the present disclosure, the communication interface 240 may perform data communication with an external server or an external electronic device under control by the at least one processor 220. For example, the communication interface 240 may perform data communication with an external server or an external electronic device by using at least one data communication method including wired LAN, wireless LAN, Wi-Fi, Bluetooth, Zigbee, Wi-Fi Direct (WFD), infrared communication (Infrared Data Association: IrDA), Bluetooth Low Energy (BLE), Near Field Communication (NFC), Wireless Broadband Internet (WiBro), Worldwide Interoperability for Microwave Access (WiMAX), Shared Wireless Access Protocol (SWAP), Wireless Gigabit Alliance (WiGig), and RF communication.

In an embodiment of the present disclosure, the at least one processor 220 may acquire an oral image 110 from an external server or an external electronic device through the communication interface 240. The at least one processor 220 may also provide a generated result image 120 or 140 to an external server or an external electronic device through the communication interface 240.

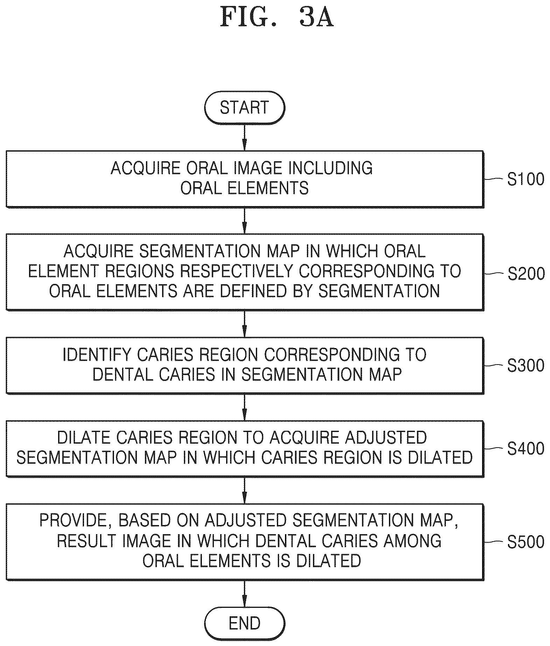

FIG. 3A is a flowchart illustrating a method of operating an oral image processing device according to an embodiment of the present disclosure.

Referring to FIGS. 1A, 1B, 2, and 3A, in an embodiment of the present disclosure, the method of operating the oral image processing device 100 may include an operation S100 of acquiring an oral image 110 including a plurality of oral elements.

In an embodiment of the present disclosure, in the operation S10 of acquiring the oral image 110, the at least one processor 220 may acquire the oral image 110 through the input/output interface 230 or the communication interface 240 by executing instructions of the image acquisition module 211.

In an embodiment of the present disclosure, the method of operating the oral image processing device 100 may include an operation S200 of acquiring a segmentation map 400 (refer to FIG. 4) in which a plurality of oral element regions respectively corresponding to the plurality of oral elements are defied by segmentation based on the oral image 110.

According to an embodiment of the present disclosure, in the operation S200 of acquiring the segmentation map 400, the at least one processor 220 may execute instructions of the segmentation map generation module 212 to acquire the segmentation map 400 in which the plurality of oral element regions respectively corresponding to the plurality of oral elements are provided through segmentation based on the oral image 110. Hereinafter, the operation of acquiring the segmentation map 400 will be described with reference to FIG. 4.

In an embodiment of the present disclosure, the method of operating the oral image processing device 100 may include an operation S300 of identifying a caries region corresponding to dental caries among the plurality of oral element regions in the segmentation map 400.

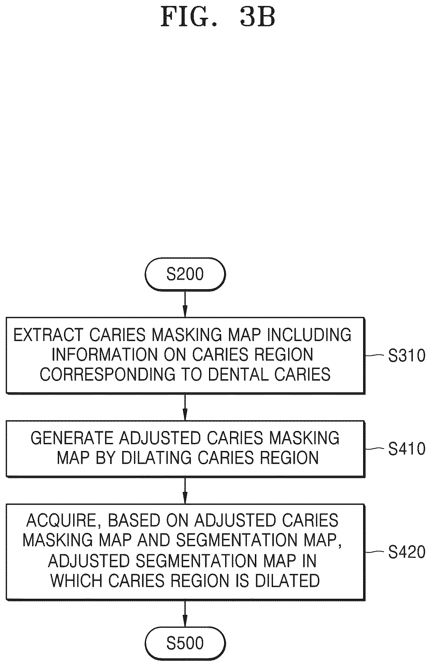

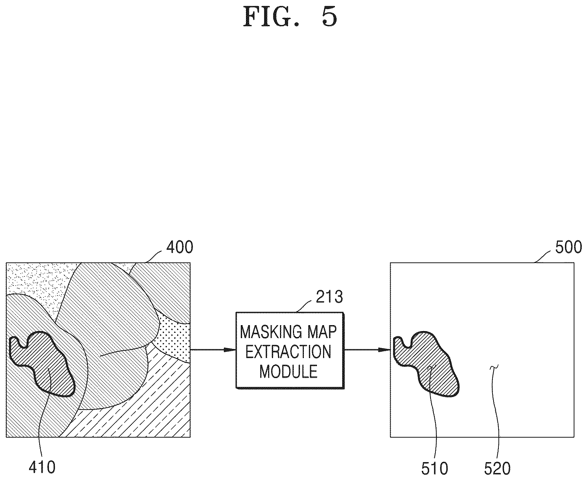

According to an embodiment of the present disclosure, in the operation S300 of identifying the caries region, the at least one processor 220 may identify the caries region corresponding to dental caries among the plurality of oral element regions in the segmentation map 400 by executing instructions of the masking map extraction module 213. Hereinafter, an operation of acquiring a caries masking map 500 will be described with reference to FIG. 3B and FIG. 5.

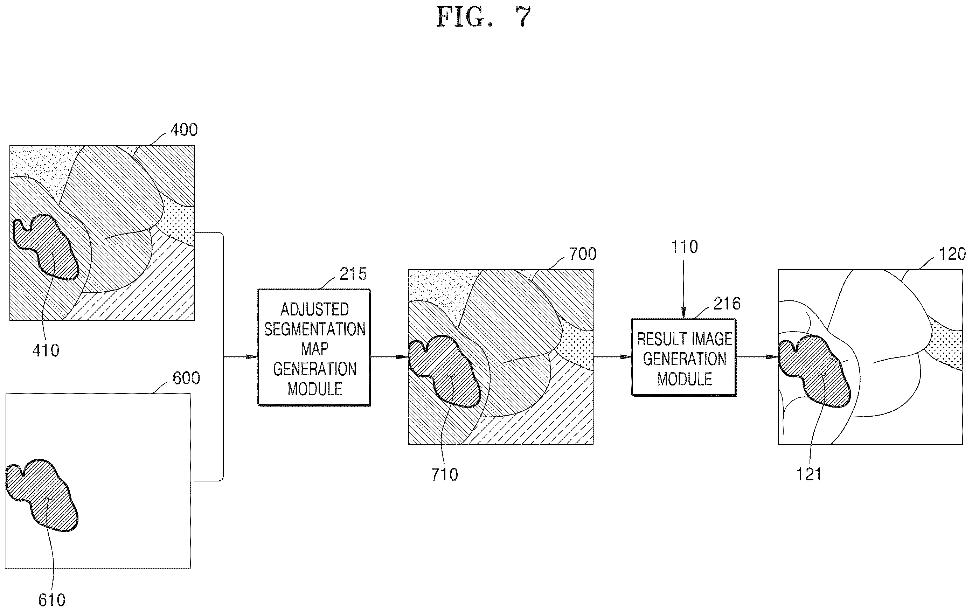

In an embodiment of the present disclosure, the method of operating the oral image processing device 100 may include an operation S400 of dilating the caries region to acquire an adjusted segmentation map 700 (refer to FIG. 7) in which the caries region among the plurality of oral element regions is dilated.

According to an embodiment of the present disclosure, in the operation S400 of acquiring the adjusted segmentation map 700 in which the caries region is dilated, the at least one processor 220 may execute instructions of the region dilation module 214 and the adjusted segmentation map generation module 215 to dilate the caries region and acquire the adjusted segmentation map 700 in which the caries region among the plurality of oral element regions is dilated. Hereinafter, the operation of acquiring the caries masking map 500 will be described with reference to FIG. 3B, FIG. 6, and FIG. 7.

In an embodiment of the present disclosure, the method of operating the oral image processing device 100 may include an operation S500 of providing, based on the adjusted segmentation map 700, a result image 120 in which the dental caries among the plurality of oral elements is displayed as the dilated caries region larger than the identified caries region.

According to an embodiment of the present disclosure, in the operation of providing the result image 120 in which the dental caries is dilated as the dilated caries region, the at least one processor 220 may execute instructions of the result image generation module 216 to acquire and provide, based on the adjusted segmentation map 700, the result image 120 in which the dental caries among the plurality of oral elements is displayed as the dilated caries region larger than the identified caries region. Hereinafter, the operation of acquiring and providing the result image 120 will be described with reference to FIG. 7.

FIG. 3B is a flowchart illustrating a method of operating an oral image processing device according to an embodiment of the present disclosure. Hereinafter, the same operations as those described with reference to FIG. 3A are denoted with the same reference numerals, and repeated descriptions thereof are omitted.

Referring to FIGS. 1A, 1B, 2, and 3B, in an embodiment of the present disclosure, the method of operating the oral image processing device 100 may include an operation S310 of extracting, from the segmentation map 400, a caries masking map 500 including information on the caries region corresponding to the dental caries among the plurality of oral element regions. In an embodiment of the present disclosure, the operation S310 of extracting the caries masking map 500 may be included in the operation S300 of identifying the caries region.

According to an embodiment of the present disclosure, in the operation S310 of extracting the caries masking map 500, the at least one processor 220 may execute instructions of the masking map extraction module 213 to extract, from the segmentation map 400, the caries masking map 500 including information on the caries region corresponding to the dental caries among the plurality of oral element regions. Hereinafter, an operation of acquiring the caries masking map 500 will be described with reference to FIG. 5.

In an embodiment of the present disclosure, the operation S310 of extracting the caries masking map 500 may be performed after the operation S200 of acquiring the segmentation map 400.

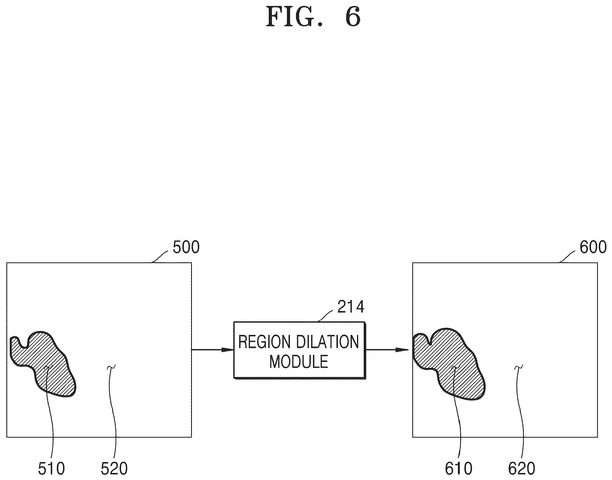

In an embodiment of the present disclosure, the method of operating the oral image processing device 100 may include an operation S410 of generating an adjusted caries masking map 600 (refer to FIG. 6) by dilating the caries region included in the caries masking map 500.

According to an embodiment of the present disclosure, in the operation S410 of generating the adjusted caries masking map 600, the at least one processor 220 may executes instructions of the region dilation module 214 to generate the adjusted caries masking map 600. Hereinafter, an operation of acquiring the adjusted caries masking map 600 will be described with reference to FIG. 6.

In an embodiment of the present disclosure, the method of operating the oral image processing device 100 may include an operation S420 of acquiring, based on the adjusted caries masking map 600 and the segmentation map 400, an adjusted segmentation map 700 in which the caries region among the plurality of oral element regions is dilated.

According to an embodiment of the present disclosure, in the operation S420 of acquiring the adjusted segmentation map 700, the at least one processor 220 may execute instructions of the adjusted segmentation map generation module 215 to acquire, based on the adjusted caries masking map 600 and the segmentation map 400, the adjusted segmentation map 700 in which the caries region among the plurality of oral element regions is dilated. Hereinafter, the operation of acquiring the adjusted segmentation map 700 will be described with reference to FIG. 7. In this case, the operation S410 of generating the adjusted caries masking map 600 and the operation S420 of acquiring the adjusted segmentation map 700 based on the adjusted caries masking map 600 and the segmentation map 400 may be included in the operation S400 of acquiring the adjusted segmentation map 700 illustrated in FIG. 3A.

In an embodiment of the present disclosure, after the operation S410 of acquiring the adjusted segmentation map 700, the operation S500 may be performed.

However, the present disclosure is not limited thereto, and the method of operating the oral image processing device 100 may further include other operations (for example, an operation of preprocessing the oral image 110 or an operation of postprocessing the result image 120, or the like) in addition to the plurality of operations illustrated in FIG. 3. In addition, some of the operations illustrated as separate operations in FIGS. 3A and 3B may be performed in a single operation.

FIG. 4 is a view illustrating an operation of acquiring a segmentation map from an oral image, according to an embodiment of the present disclosure.

Referring to FIGS. 2, 3, and 4, in an embodiment of the present disclosure, the oral image processing device 100 may generate the segmentation map 400 by segmenting the oral image 110 through the segmentation map generation module 212 into a plurality of oral element regions respectively corresponding to a plurality of oral elements.

In an embodiment of the present disclosure, the segmentation map generation module 212 may include instructions or program code related to an operation or function for segmenting the oral image 110 into a plurality of oral element regions respectively corresponding to a plurality of oral elements.

In an embodiment of the present disclosure, the segmentation map generation module 212 may include an algorithm for segmenting the oral image 110 into a plurality of oral elements. The segmentation map generation module 212 may include an algorithm for segmenting the oral image 110 into a plurality of oral elements through an operation of classifying a plurality of pixels of the oral image 110 into one or more categories according to characteristics of the plurality of pixels.

In an embodiment of the present disclosure, the resolution of the plurality of pixels included in the segmentation map 400 may be the same as the resolution of the plurality of pixels included in the oral image 110.

In an embodiment of the present disclosure, the plurality of oral elements included in the oral image 110 may be assigned to a plurality of oral element regions respectively corresponding thereto through the segmentation map generation module 212.

In an embodiment of the present disclosure, each of the plurality of pixels included in the segmentation map 400 may include category information about a corresponding oral element.

In an embodiment of the present disclosure, one or more pixels including the same category information among the plurality of pixels included in the segmentation map 400 may be included in the same oral element region. Accordingly, the plurality of oral element regions included in the oral image 110 may be assigned respectively the plurality of oral element regions corresponding thereto through the segmentation map generation module 212. In an embodiment of the present disclosure, a caries region 410 corresponding to dental caries among the plurality of oral element regions included in the oral image 110 may be defined using the segmentation map generation module 212.

However, the present disclosure is not limited thereto, and category information values used to classify the plurality of oral elements may vary according to an algorithm, setting values, or a training method of the segmentation map generation module 212. In addition, as the number of types of oral elements included in the oral image 110 increases, the number of types of category information included in the segmentation map 400 may also increase.

In an embodiment of the present disclosure, the segmentation map generation module 212 may include an artificial intelligence model. The artificial intelligence model included in the segmentation map generation module 212 may include a machine learning model or a deep learning model. In an embodiment of the present disclosure, the artificial intelligence model included in the segmentation map generation module 212 may be an artificial intelligence model trained to receive the oral image 110 as an input and infer the segmentation map 400 in which a plurality of oral element regions respectively corresponding to the plurality of oral elements included in the oral image 110 are defined.

In an embodiment of the present disclosure, the artificial intelligence model included in the segmentation map generation module 212 may include a convolutional neural network (CNN), a recurrent neural network (RNN), a fully convolutional network (FCN), SegNet, U-Net, DeepLab, a proportional-integral-derivative net (PIDNet), or the like. However, the artificial intelligence model included in the segmentation map generation module 212 of the present disclosure is not limited to the listed examples.

In an embodiment of the present disclosure, the artificial intelligence model included in the segmentation map generation module 212 may be a model pretrained using data sets each including a training oral image including a plurality of oral elements and labeling information in which the plurality of oral elements included in the training oral image are labeled.

In an embodiment of the present disclosure, types of category information included in the segmentation map 400 inferred through the segmentation map generation module 212 may vary according to the labeling information included in the data sets. In an embodiment of the present disclosure, when the segmentation map generation module 212 is trained using data sets including labeling information labeled only as "tooth" and "dental caries," the inferred segmentation map 400 may selectively classify the plurality of oral elements included in the oral image 110 into a tooth region corresponding to a "tooth" and a caries region corresponding to "dental caries."

However, the present disclosure is not limited thereto, and types of category information included in the inferred segmentation map 400 may also be determined by changing parameters of the segmentation map generation module 212.

FIG. 5 is a view illustrating an operation of extracting a caries masking map including information on a caries region corresponding to dental caries, according to an embodiment of the present disclosure.

Referring to FIGS. 2, 3, and 4, in an embodiment of the present disclosure, the oral image processing device 100 may extract, from the segmentation map 400, a caries masking map 500 including information on the caries region 410 corresponding to dental caries among the plurality of oral element regions through the masking map extraction module 213.

In an embodiment of the present disclosure, the masking map extraction module 213 may include instructions or program code related to an operation or function for identifying a region of interest (ROI) among the plurality of oral element regions included in the segmentation map 400 and extracting only the ROI.

In an embodiment of the present disclosure, the masking map extraction module 213 may include instructions or program code related to an operation or function for extracting the ROI among the plurality of oral element regions included in the segmentation map 400 and masking the remaining regions.

In an embodiment of the present disclosure, the masking map extraction module 213 may include instructions or program code related to an operation or function for generating a masking map by processing the ROI as "1" and the remaining regions as "0."

In an embodiment of the present disclosure, the masking map extraction module 213 may set the caries region 410 among the plurality of oral element regions as an ROI 510. The masking map extraction module 213 may include instructions or program code related to an operation or function for extracting, from the segmentation map 400, the caries masking map 500 in which the ROI 510 has a value of "1" and a remaining region 520 has a value of "0." The caries masking map 500 may include information on the ROI 510 having a value of "1."

FIG. 6 is a view illustrating an operation of dilating a caries region, according to an embodiment of the present disclosure.

Referring to FIGS. 2, 3, and 6, in an embodiment of the present disclosure, the oral image processing device 100 may generate an adjusted caries masking map 600 through the region dilation module 214 by dilating the ROI 510 that is included in the caries masking map 500 and corresponds to the caries region 410.

In an embodiment of the present disclosure, the region dilation module 214 may include instructions or program code related to an operation or function for dilating the ROI 510 included in the caries masking map 500 to generate an adjusted caries region 610.

In an embodiment of the present disclosure, the region dilation module 214 may include instructions or program code related to an operation or function for generating the adjusted caries region 610 by dilating the ROI 510 included in the masking map 500 through a morphology operation. The region dilation module 214 may include instructions or program code related to an operation or function for dilating the caries masking map 500, which is in the form of binary data. The region dilation module 214 may dilate a boundary portion of the ROI 510 to generate the adjusted caries region 610.

In an embodiment of the present disclosure, the ROI 510 may be dilated as the adjusted caries region 610 by the region dilation module 214. The area of the adjusted caries region 610 may be greater than the area of the ROI 510. In an embodiment of the present disclosure, the adjusted caries masking map 600 may include the adjusted caries region 610 and a remaining region 620. In an embodiment of the present disclosure, the adjusted caries region 610 of the adjusted caries masking map 600 may have a value of "1," and the remaining region 620 of the adjusted caries masking map 600 may have a value of "0."

FIG. 7 is a view illustrating an operation of acquiring an adjusted segmentation map in which a caries region is dilated, according to an embodiment of the present disclosure.

Referring to FIGS. 2, 3, and 7, in an embodiment of the present disclosure, the oral image processing device 100 may generate an adjusted segmentation map 700 in which the caries region 410 among the plurality of oral elements is dilated through the adjusted segmentation map generation module 215 based on the adjusted caries masking map 600 and the segmentation map 400

In an embodiment of the present disclosure, the adjusted segmentation map generation module 215 may include instructions or program code related to an operation or function for generating, based on the segmentation map 400 and the adjusted caries masking map 600, the adjusted segmentation map 700 in which the adjusted caries region 610 is reflected.

In an embodiment of the present disclosure, the adjusted segmentation map generation module 215 may include instructions or program code related to an operation or function for generating the adjusted segmentation map 700 by applying information included in the adjusted caries masking map 600 to the segmentation map 400. The adjusted segmentation map generation module 215 may include instructions or program code related to an operation or function for reflecting, in the segmentation map 400, a region including information corresponding to "1" in the adjusted caries masking map 600, and not reflecting, in the segmentation map 400, a region including information corresponding to "0" in the adjusted caries masking map 600.

Accordingly, the adjusted segmentation map 700 may include the adjusted caries region 610 and the remaining regions included in the segmentation map 400. In an embodiment of the present disclosure, the adjusted segmentation map 700 may include a dilated caries region 710 corresponding to the adjusted caries region 610.

In an embodiment of the present disclosure, the oral image processing device 100 may generate a result image 120 based on the adjusted segmentation map 700 through the result image generation module 216. In the result image 120, a specific oral element among a plurality of oral elements is displayed as a dilated region that is larger than a corresponding region identified through the segmentation map generation module 212.

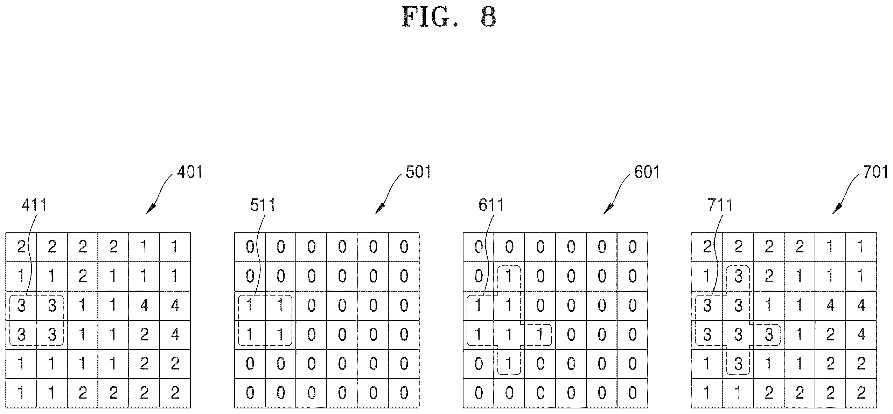

The oral image processing device 100 may generate the result image 120, through the result image generation module 216 based on the adjusted segmentation map 700 and the oral image 110, in which a specific oral element among the plurality of oral elements is displayed as a dilated region that is larger than a corresponding region identified through the segmentation map generation module 212. In this case, the result image 120 may be a two-dimensional image. However, the present disclosure is not limited thereto, and as illustrated below with reference to in FIGS. 8 to 10, the result image 120 may be a three-dimensional image.

In this case, the specific oral element may be dental caries. The oral image processing device 100 may generate, through the result image generation module 216, the result image 120 in which a caries region, identified as having dental caries among the plurality of oral elements, is displayed as a dilated caries region 121.

In an embodiment of the present disclosure, the oral image processing device 100 may provide, through the display 200, the result image 120 generated using the result image generation module 216. In addition, the oral image processing device 100 may provide the result image 120 to an external electronic device that is connected through the input/output interface 230 or the communication interface 240. The oral image processing device 100 may also provide the result image 120 to an external server that is connected through the communication interface 240.

In an embodiment of the present disclosure, the result image generation module 216 may include instructions or program code related to an operation or function for displaying or emphasizing a dilated specific region included in the oral image 110 in a different color by using the adjusted segmentation map 700. In an embodiment of the present disclosure, the result image generation module 216 may include instructions or program code related to an operation or function for emphasizing and displaying a region of the oral image 110 that corresponds to a dilated region among the plurality of oral element regions of the adjusted segmentation map 700.

In an embodiment of the present disclosure, the result image generation module 216 may include instructions or program code related to an operation or function for generating the result image 120 in which the dilated caries region 710 included in the adjusted segmentation map 700 is emphasized and finally displayed as the caries region 121. In this case, the final caries region 121 may be displayed in a preselected color.

In an embodiment of the present disclosure, the oral image processing device 100 may provide the result image 120 including the dilated region 121 by dilating a region of a specific oral element (for example, dental caries 111) included in the acquired oral image 110. The oral image processing device 100 may enhance visibility for a user by displaying the dilated region 121, included in the result image 120 and corresponding to a specific oral element, in a different color.

In an embodiment of the present disclosure, the oral image processing device 100 may enable a user, for example, a dentist, to recognize a specific oral element such as dental caries in the oral cavity even when the oral element is small or obscured by another oral element. Through this, the dentist may easily establish a treatment plan for dental caries by using the oral image processing device 100.

For example, dental caries having a point-like shape on the surface of a tooth may have progressed considerably and may thus be larger within the tooth than on the surface of the tooth. In this case, when a dentist performs an examination using the oral image 110 or a result image in which a caries region is not dilated, the dentist may fail to detect the dental caries or may not properly determine the degree of progression of dental caries, thereby having difficulty in establishing a treatment plan.

In the result image 120 provided by the oral image processing device 100 according to an embodiment of the present disclosure, a region corresponding to dental caries included in the oral image 110 is dilated. Thus, compared to a case in which a dentist performs an examination using the oral image 110, when the dentist performs an examination using the result image 120, the probability of recognizing and detecting the presence, location, and shape of dental caries of a patient may be increased. Accordingly, it may become easier for the dentist to establish a treatment plan for the dental caries of the patient.

FIG. 8 is a view illustrating a segmentation map, a caries masking map, an adjusted caries masking map, and an adjusted segmentation map according to an embodiment of the present disclosure.

Referring to FIGS. 4, 5, 6, 7, and 8, FIG. 8 illustrates first to fourth pixel maps 401, 501, 601, and 701 respectively for describing the segmentation map 400, the caries masking map 500, the adjusted caries masking map 600, and the adjusted segmentation map 700, according to an embodiment of the present disclosure.

Referring to FIG. 8, for ease of description, each of the first to fourth pixel maps 401, 501, 601, and 701 is illustrated as having 6*6 pixels, but the present disclosure is not limited thereto. The resolution of the plurality of pixels included in each of the first to fourth pixel maps 401, 501, 601, and 701 may be the same as the resolution of the plurality of pixels included in the oral image 110.

In an embodiment of the present disclosure, the first pixel map 401 is a view for describing the segmentation map 400. The second pixel map 501 is a view for describing the caries masking map 500. The third pixel map 601 is a view for describing the adjusted caries masking map 600. The fourth pixel map 701 is a view for describing the adjusted segmentation map 700.

In an embodiment of the present disclosure, the segmentation map 400 may be generated using the segmentation map generation module 212 by segmenting the oral image 110 into a plurality of oral element regions respectively corresponding to a plurality of oral elements.

In an embodiment of the present disclosure, referring to the first pixel map 401, each of the plurality of pixels included in the segmentation map 400 may include segmentation category information on a corresponding oral element.

In an embodiment of the present disclosure, the segmentation map generation module 212 may classify "teeth," among the oral elements included in the oral image 110, as a category corresponding to "1." The segmentation map generation module 212 may classify "gums," among the oral elements included in the oral image 110, as a category corresponding to "2." The segmentation map generation module 212 may classify "dental caries," among the oral elements included in the oral image 110, as a category corresponding to "3." The segmentation map generation module 212 may classify "prosthesis," among the oral elements included in the oral image 110, as a category corresponding to "4."

In an embodiment of the present disclosure, the caries masking map 500 may be generated by extracting, using the masking map extraction module 213, the caries region 410 as the ROI 510 from among the plurality of oral element regions included in the segmentation map 400, and masking the remaining regions.

In an embodiment of the present disclosure, referring to the second pixel map 501, an ROI 511 corresponding to a caries region 411 of the first pixel map 401 may be extracted as "1," and the remaining regions may be masked as "0."

In an embodiment of the present disclosure, the adjusted caries masking map 600 may be generated using the region dilation module 214 by dilating the ROI 510 included in the caries masking map 500.

In an embodiment of the present disclosure, referring to the third pixel map 601, an adjusted caries region 611 obtained by dilating the ROI 511 included in the second pixel map 501 may have a value of "1," and the remaining regions may have a value of "0." In this case, the area of the adjusted caries region 611 may be greater than the area of the ROI 511.

In an embodiment of the present disclosure, the adjusted segmentation map 700 may be generated by reflecting the adjusted caries region 610 using the adjusted segmentation map generation module 215 based on the adjusted caries masking map 600 and the segmentation map 400.

In an embodiment of the present disclosure, referring to the fourth pixel map 701, the adjusted caries region 611 included in the third pixel map 601 is reflected in the first pixel map 401, such that a region of the first pixel map 401 corresponding to the adjusted caries region 611 may be assigned "3" and the remaining regions of the first pixel map 401 may have original segmentation information.

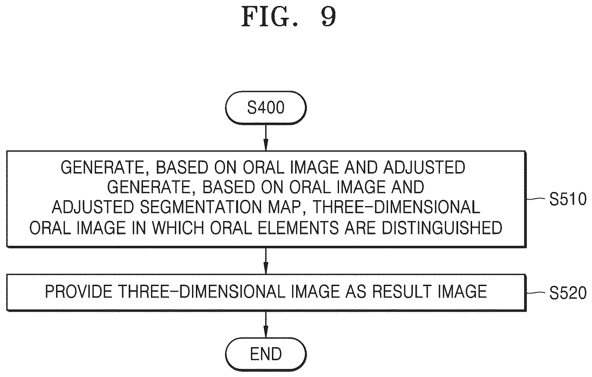

FIG. 9 is a flowchart illustrating an operation of generating a three-dimensional oral image based on an oral image and an adjusted segmentation map, according to an embodiment of the present disclosure. Hereinafter, operations identical to those described with reference to FIG. 3A are denoted by the same reference numerals, and repeated descriptions are omitted.

Referring to FIGS. 1B, 2, 3A, and 9, in an embodiment of the present disclosure, the method of operating the oral image processing device 100 may include an operation S510 of generating, based on the oral image 110 and the adjusted segmentation map 700 (refer to FIG. 7), a three-dimensional oral image 140 in which a specific oral element (for example, a tooth element 131) among a plurality of oral elements is displayed as a dilated region (for example, a dilated caries region 141) larger than a corresponding region identified in the segmentation map.

In an embodiment of the present disclosure, the method of operating the oral image processing device 100 may include an operation S520 of providing the three-dimensional oral image 140 as a result image.

In an embodiment of the present disclosure, the operation S510 of generating the three-dimensional oral image 140 and the operation S520 of providing the three-dimensional oral image 140 as a result image may be included in the operation S500 of providing a result image.

In an embodiment of the present disclosure, the operation S510 of generating the three-dimensional oral image 140 may be performed after the operation S400 of acquiring the adjusted segmentation map 700.

According to an embodiment of the present disclosure, in the operation S510 of generating the three-dimensional oral image 140, the at least one processor 220 may execute instructions of the result image generation module 216 to generate, based on the oral image 110 and the adjusted segmentation map 700, the three-dimensional oral image 140 in which the dental caries 131 among the plurality of oral elements is displayed as the dilated caries region 141 larger than the identified caries region.

In an embodiment of the present disclosure, the result image generation module 216 may include instructions or program code related to an operation or function for extracting, based on the oral image 110 and the adjusted segmentation map 700, feature points of each of the plurality of oral elements included in the oral image 110. The result image generation module 216 may include instructions or program code for three-dimensionally modeling a plurality of oral elements included in each of a plurality of oral images 130 acquired at different poses by a triangulation method using disparity, that is, using a positional difference between feature points of the plurality of oral elements. In addition, the result image generation module 216 may include instructions or program code for performing three-dimensionally modeling by a confocal method using results acquired using a laser.

However, the present disclosure is not limited thereto. In an embodiment of the present disclosure, the result image generation module 216 may include an artificial intelligence model that is pretrained to generate a three-dimensional oral image 140 by receiving the oral image 110 and the adjusted segmentation map 700 as inputs.

In an embodiment of the present disclosure, the result image generation module 216 may include an artificial intelligence model that is pretrained to infer depth data by receiving the oral image 110 as an input. The result image generation module 216 may include an artificial intelligence model that is pretrained to infer a three-dimensional oral image 140 including voxels or meshes by receiving inferred depth data, the oral image 110, and the adjusted segmentation map 700 as inputs.

In an embodiment of the present disclosure, the artificial intelligence model included in the result image generation module 216 may include a convolutional neural network (CNN), a variational auto encoder (VAE), a generative adversarial network (GAN), a transformer, or the like, but is not limited to any one of them.

According to an embodiment of the present disclosure, in the operation S520 of providing the three-dimensional oral image 140 as a result image, the at least one processor 220 may provide the three-dimensional oral image 140 as a result image.

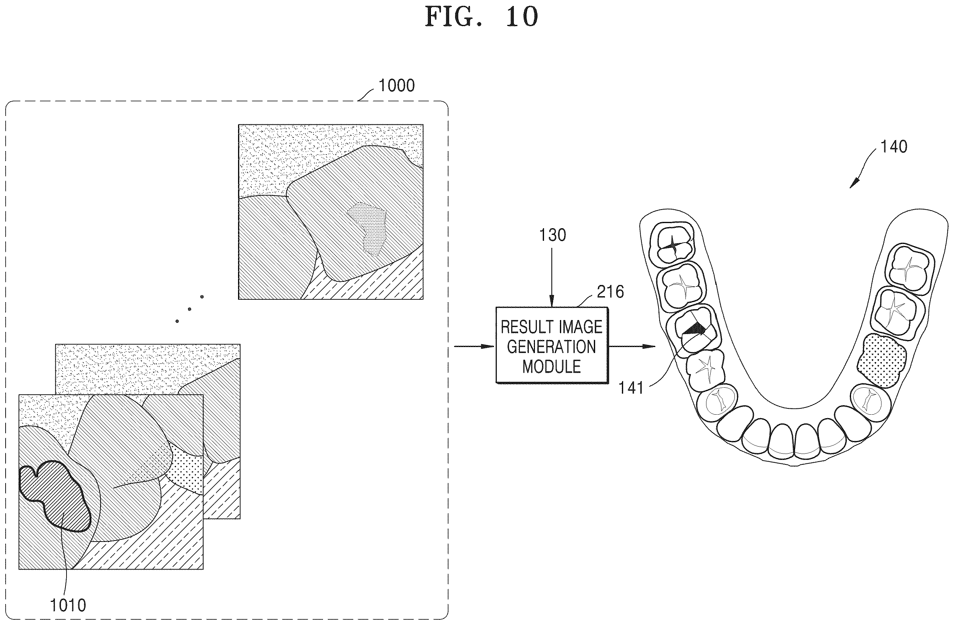

FIG. 10 is a view illustrating an operation of generating a three-dimensional oral image based on a plurality of adjusted segmentation maps and a plurality of oral images, according to an embodiment of the present disclosure.

Referring to FIGS. 1B, 2, 3A, 3B, and 10, in an embodiment of the present disclosure, the oral image processing device 100 may provide a three-dimensional oral image 140 generated based on a plurality of oral images 130. In an embodiment of the present disclosure, as the plurality of oral images 130 are acquired in the operation S100 of acquiring an oral image 110, the oral image processing device 100 may generate the three-dimensional oral image 140 by using the plurality of oral images 130.

In an embodiment of the present disclosure, the operation S200 of acquiring a segmentation map 400 (refer to FIG. 4) may include acquiring, based on the plurality of oral images 130, a plurality of segmentation maps in which a plurality of oral element regions respectively corresponding to a plurality of oral elements included in each of the plurality of oral images 130 are defined by segmentation.

In an embodiment of the present disclosure, the operation illustrated in FIG. 4 may refer to an operation of acquiring one of the plurality of segmentation maps, based on one of the plurality of oral images 130. In an embodiment of the present disclosure, the oral image processing device 100 may perform the operation illustrated in FIG. 4 for each of the plurality of oral images 130, thereby acquiring the plurality of segmentation maps corresponding to the plurality of oral images 130.

In an embodiment of the present disclosure, the at least one processor 220 may execute instructions of the segmentation map generation module 212 to acquire, based on the plurality of oral images 130, a plurality of segmentation maps in which a plurality of oral element regions respectively corresponding to the plurality of oral elements are defined by segmentation.

In an embodiment of the present disclosure, the operation S310 of acquiring a caries masking map 500 (refer to FIG. 5) may include extracting, respectively from the plurality of segmentation maps, a plurality of caries masking maps each including information on a caries region 510 corresponding to dental caries among the plurality of oral elements.

In an embodiment of the present disclosure, the operation illustrated in FIG. 5 may refer to an operation of extracting, based on one of the plurality of segmentation maps, one of the plurality of caries masking maps each including information on a caries region corresponding to dental caries among the plurality of oral elements.

In one embodiment, the oral image processing device 100 may perform the operation illustrated in FIG. 5 for each of the plurality of segmentation maps, thereby extracting the plurality of caries masking maps corresponding to the plurality of segmentation maps.

In an embodiment of the present disclosure, the at least one processor 220 may execute instructions of the masking map extraction module 213 to extract, respectively from the plurality of segmentation maps, a plurality of caries masking maps each including information on a caries region corresponding to dental caries among the plurality of oral element regions.

In an embodiment of the present disclosure, the operation S410 of generating an adjusted caries masking map 600 (refer to FIG. 6) may include generating a plurality of adjusted caries masking maps by dilating a caries region included in each of the plurality of caries masking maps.

In an embodiment of the present disclosure, the operation illustrated in FIG. 6 may refer to an operation of generating one of the plurality of adjusted caries masking maps by dilating the caries region included in one of the plurality of caries masking maps. A dilated caries masking region 1010 included in each of the plurality of adjusted caries masking maps may be larger than the caries region included in each of the plurality of caries masking maps.

In one embodiment, the oral image processing device 100 may perform the operation illustrated in FIG. 6 for each of the plurality of caries masking maps, thereby extracting the plurality of adjusted caries masking maps respectively corresponding to the plurality of caries masking maps.

In an embodiment of the present disclosure, the at least one processor 220 may execute instructions of the region dilation module 214 to generate a plurality of adjusted caries masking maps by dilating a caries region included in each of the plurality of caries masking maps.

In an embodiment of the present disclosure, the operation S430 of acquiring an adjusted segmentation map (700, refer to FIG. 7) may include acquiring, based on each of the plurality of adjusted caries masking maps and each of the plurality of segmentation maps corresponding to the plurality of adjusted caries masking maps, a plurality of adjusted segmentation maps in which caries regions among a plurality of oral regions are dilated.

In an embodiment of the present disclosure, the operation illustrated in FIG. 7 may refer to an operation of generating one of the plurality of adjusted segmentation maps, based on one of the plurality of adjusted caries masking maps and one of the plurality of segmentation maps.

In one embodiment, the oral image processing device 100 may perform the operation illustrated in FIG. 7 for each of the plurality of adjusted caries masking maps and each of the plurality of segmentation maps, thereby generating the plurality of adjusted segmentation maps corresponding thereto.

In an embodiment of the present disclosure, the at least one processor 220 may execute instructions of the adjusted segmentation map generation module 215 to acquire, based on each of the plurality of adjusted caries masking maps and each corresponding one of the plurality of segmentation maps, a plurality of adjusted segmentation maps in which caries regions among a plurality of oral regions are dilated.

Referring to FIGS. 9 and 10, in an embodiment of the present disclosure, the operation S510 of generating a three-dimensional oral image 140 may include generating a three-dimensional oral image 140 based on a plurality of adjusted segmentation maps 1000 and a plurality of oral images 130.

In an embodiment of the present disclosure, the oral image processing device 100 may generate, using the result image generation module 216 based on the plurality of adjusted segmentation maps 1000 and the plurality of oral images 130, a three-dimensional oral image 140 in which a specific oral element (for example, dental caries) among a plurality of oral elements is displayed as a dilated region (for example, a dilated caries region 141) larger than an identified region. In this case, the plurality of oral elements included in the three-dimensional oral image 140 may correspond to the plurality of oral elements included in each of the plurality of oral images 130.

In an embodiment of the present disclosure, the result image generation module 216 may include instructions or program code related to an operation or function for displaying a region, which is identified as dental caries among a plurality of oral elements in each of the plurality of oral images 130 by using the plurality of adjusted segmentation maps 1000, as a dilated caries region.

In an embodiment of the present disclosure, the result image generation module 216 may include a triangular module, a confocal module, or an artificial intelligence model that generates a point cloud forming the three-dimensional oral image 140 by using estimated poses of the plurality of oral images 130 and extracted feature points of a plurality of oral elements included in each of the plurality of oral images 130.

In an embodiment of the present disclosure, the result image generation module 216 may include instructions or program code related to an operation or function for generating the three-dimensional oral image 140 by converting the generated point cloud into voxels or meshes. However, the present disclosure is not limited thereto, and the result image generation module 216 may include instructions or program code related to operations or functions that may be performed to generate, based on the plurality of adjusted segmentation maps 1000 and the plurality of oral images 130, the three-dimensional oral image 140 in which a plurality of oral elements are distinguished from each other.

In an embodiment of the present disclosure, the operation S510 of generating the three-dimensional oral image 140 may include generating a plurality of point clouds based on the plurality of oral images 130.

The at least one processor 220 may generate the plurality of point clouds based on the plurality of oral images 130 by executing instructions of the result image generation module 216.

In an embodiment of the present disclosure, the operation S510 of generating the three-dimensional oral image 140 may include generating, based on a plurality of point clouds and a plurality of adjusted segmentation maps 900, a three-dimensional oral image 140 including a plurality of voxels including the plurality of point clouds.

The at least one processor 220 may execute instructions of the result image generation module 216 to generate, based on a plurality of point clouds and a plurality of adjusted segmentation maps 900, a three-dimensional oral image 140 including a plurality of voxels including the plurality of point clouds.

In an embodiment of the present disclosure, each of the plurality of voxels may include at least one point cloud. Information on each of the plurality of voxels may be determined based on information on at least one point cloud included in the corresponding voxel, and the information on the at least one point cloud may include, for example, types, colors, or textures of oral elements.

In an embodiment of the present disclosure, the oral image processing device 100 may generate the three-dimensional oral image 140 including the plurality of voxels, and thus, a time required to generate the three-dimensional oral image 140 from the plurality of oral images 130 may be reduced. Therefore, the three-dimensional oral image 140 generated from images acquired by scanning the oral cavity of a patient using an oral scanner may be quickly provided to a dentist.

However, the present disclosure is not limited thereto. The method of operating the oral image processing device 100 may further include post-processing the three-dimensional oral image 140 including the plurality of voxels to generate a post-processed three-dimensional oral image including a plurality of meshes. The at least one processor 220 may post-process the three-dimensional oral image 140 including the plurality of voxels to generate the post-processed three-dimensional oral image including the plurality of meshes.

In an embodiment of the present disclosure, the method of operating the oral image processing device 100 may further include generating a plurality of meshes including information on the three-dimensional oral image 140 by using at least one of the plurality of voxels and the plurality of point clouds.

The at least one processor 220 may generate the plurality of meshes including information on the three-dimensional oral image 140 by using at least one of the plurality of voxels forming the three-dimensional oral image 140 and the plurality of point clouds. In this case, information regarding the three-dimensional oral image 140, which is included in the plurality of voxels, may be included at vertices of the plurality of meshes. In addition, information regarding the three-dimensional oral image 140, which is included in the plurality of voxels, may be converted into separate texture information, and the texture information may be mapped to the plurality of meshes.

In an embodiment of the present disclosure, the method of operating the oral image processing device 100 may include generating the post-processed three-dimensional oral image by using the plurality of meshes. The at least one processor 220 may generate the post-processed three-dimensional oral image by using the plurality of meshes.

In an embodiment of the present disclosure, the oral image processing device 100 may post-process the three-dimensional oral image 140 including the plurality of voxels to generate the post-processed three-dimensional oral image including the plurality of meshes, and thus, the rendering efficiency of three-dimensional oral images may be improved. In addition, the size of post-processed three-dimensional oral images may be smaller than the size of three-dimensional oral images, and thus, storage capacity required to store data may be minimized. In addition, when using result images generated by the oral image processing device 100 of the present disclosure in another application (for example, an editing application, a visualization application, an animation application, or the like), the compatibility of post-processed three-dimensional oral images including a plurality of meshes may be increased.

In an embodiment of the present disclosure, when providing the generated three-dimensional oral image to an external electronic device or an external server through the input/output interface 230 or the communication interface 240, the oral image processing device 100 may provide the three-dimensional oral image after processing the three-dimensional oral image to include a plurality of meshes.

In an embodiment of the present disclosure, the oral image processing device 100 may assign a first weight to the dilated caries region 710 among the plurality of oral regions included in the adjusted segmentation map 700 (refer to FIG. 7), and assign a second weight to the remaining regions among the plurality of oral regions. Among the plurality of oral regions, the dilated caries region 710 may have the first weight, and the remaining regions may have the second weight.

In an embodiment of the present disclosure, the oral image processing device 100 may assign the first weight to a dilated caries region 1010 among the plurality of oral regions included in each of the plurality of adjusted segmentation maps 1000, and assign the second weight to the remaining regions among the plurality of oral regions. Among the plurality of oral regions included in each of the plurality of adjusted segmentation maps 1000, the dilated caries region 1010 may have the first weight, and the remaining regions may have the second weight.

In an embodiment of the present disclosure, the first weight may be greater than the second weight. Specifically, the first weight may be "3," and the second weight may be "1." However, the present disclosure is not limited thereto. The ratio of the first weight to the second weight may be changed.

In an embodiment of the present disclosure, when generating the three-dimensional oral image 140 in which a specific oral element among a plurality of oral elements is displayed as a dilated region larger than an originally identified region, weights assigned to regions of each of the plurality of adjusted segmentation maps 1000 may be comprehensively used.

According to an embodiment of the present disclosure, in the operation S510 of generating the three-dimensional oral image 140, the three-dimensional oral image 140 may be generated by integrating all of the plurality of adjusted segmentation maps 1000 such that a region having the first weight greater than the second weight may be displayed as a dilated caries region 141 corresponding to dental caries among the plurality of oral regions.

In an embodiment of the present disclosure, the at least one processor 220 may execute instructions of the result image generation module 216 to generate the three-dimensional oral image 140 by integrating all of the plurality of adjusted segmentation maps 1000 such that a region having the first weight greater than the second weight may be displayed as a dilated caries region 141 corresponding to dental caries among the plurality of oral regions.

Specifically, in the operation S510 of generating the three-dimensional oral image 140, the at least one processor 220 may generate the three-dimensional oral image 140 by summing all of the plurality of adjusted segmentation maps 1000 such that a region having the first weight greater than the second weight may be displayed as a dilated caries region 141 corresponding to dental caries among the plurality of oral regions.

Each of the plurality of oral images 130 may include different oral elements or different shapes of the same oral element depending on a photographing position within the oral cavity, a photographing angle, ambient illumination, or the like. Accordingly, the position or shape of a region (for example, a caries region) corresponding to a specific oral element (for example, dental caries) included in the plurality of adjusted segmentation maps acquired using the plurality of oral images 130 may vary.

For example, the same oral element may be classified as a caries region in one adjusted segmentation map and as another region (for example, a tooth region or a gum region) in another adjusted segmentation map. In this case, rather than intactly using the plurality of adjusted segmentation maps, different weights may be applied to the plurality of oral regions included in each of the adjusted segmentation maps to generate the three-dimensional oral image 140 in which a caries region is preferentially distinguished. Specifically, a weight for the dilated caries region 1010 included in each of the plurality of adjusted segmentation maps may be greater than a weight for the remaining regions.

Therefore, when the same oral element is classified as a dilated caries region 1010 in one adjusted segmentation map and as another region in another adjusted segmentation map, the at least one processor 220 may determine, based on the result of adding the two adjusted segmentation maps, that the oral element corresponds to a dilated caries region 1010 because a weight indicating that the oral element corresponds to a dilated caries region 1010 is greater than a weight indicating that the oral element corresponds to another region. Therefore, the at least one processor 220 may generate the three-dimensional oral image 140 in which the oral element is displayed as the dilated caries region 141 corresponding to dental caries.

For example, when a plurality of adjusted segmentation maps are generated based on a plurality of oral images obtained by photographing the same oral element, the at least one processor 220 may count the first weight and the second weight assigned to the same pixel in the plurality of adjusted segmentation maps, and may determine whether the sum of the first weights is greater than the sum of the second weights, thereby determining whether the pixel corresponds to a dilated caries region 1010.

Accordingly, the oral image processing device 100 may provide, to a user, the three-dimensional oral image 140 in which a region corresponding to an oral element that the user wants to emphasize, for example, dental caries, is shown on a dilated scale, thereby enhancing visibility for the user. In addition, the oral image processing device 100 may provide, to the user, the three-dimensional oral image 140 in which dental caries that may be omitted depending on photographing conditions or performance of an oral scanner is clearly displayed.

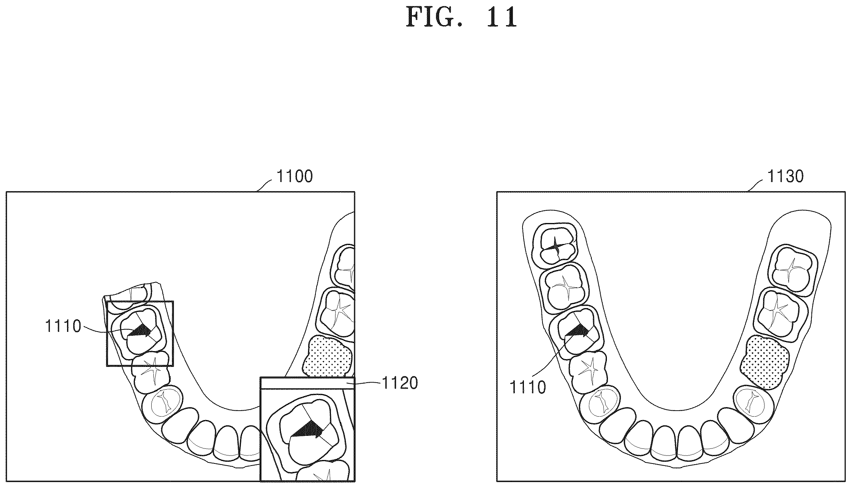

FIG. 11 is a view illustrating an operation of providing a three-dimensional oral image, according to an embodiment of the present disclosure.

Referring to FIGS. 1B, 2, and 11, in an embodiment of the present disclosure, an oral image 110 provided to the oral image processing device 100 may be a fully captured image owing to completion of scanning a patient's oral cavity using an oral scanner or the like. However, the present disclosure is not limited thereto, and the oral image 110 may be an image acquired during scanning of the patient's oral cavity.

Referring to FIG. 11, a first result image 1100 and a second result image 1130 are illustrated according to an embodiment of the present disclosure.

In an embodiment of the present disclosure, the first result image 1100 may be a result image in the case in which an oral image 110 acquired during scanning the patient's oral cavity is provided to the oral image processing device 100. In an embodiment of the present disclosure, the first result image 1100 may be updated in response to a new oral image that is provided to the oral image processing device 100 while the patient's oral cavity is continuously scanned. In an embodiment of the present disclosure, the first result image 1100 may be a three-dimensional representation of a portion of the patient's oral cavity that is completely scanned using the oral scanner. The first result image 1100 may be implemented as a three-dimensional oral image.

In an embodiment of the present disclosure, the second result image 1130 may be a result image in the case in which an oral image 110 fully captured after completion of scanning the patient's oral cavity is provided to the oral image processing device 100. In an embodiment of the present disclosure, the second result image 1130 may be a three-dimensional representation of the patient's oral cavity that is completely scanned using the oral scanner. The second result image 1130 may be implemented as a three-dimensional oral image.

In an embodiment of the present disclosure, the oral image processing device 100 may display a dilated caries region 1110 in each of the first result image 1100 and the second result image 1130. In this case, the oral image processing device 100 may display the dilated caries region 1110 in a preselected color to emphasize the dilated caries region 1110 for a user. In addition, the oral image processing device 100 may enhance visibility for the user by enlarging the dilated caries region 1110 compared with the oral image 110.

In an embodiment of the present disclosure, the oral image processing device 100 may provide, through the display 200, an interface including the first result image 1100 or the second result image 1130. In this case, the oral image processing device 100 may provide the interface with an interface 1120, and the interface 1120 may display an oral image captured using the oral scanner and corresponding to a specific region of the first result image 1100 or the second result image 1130.

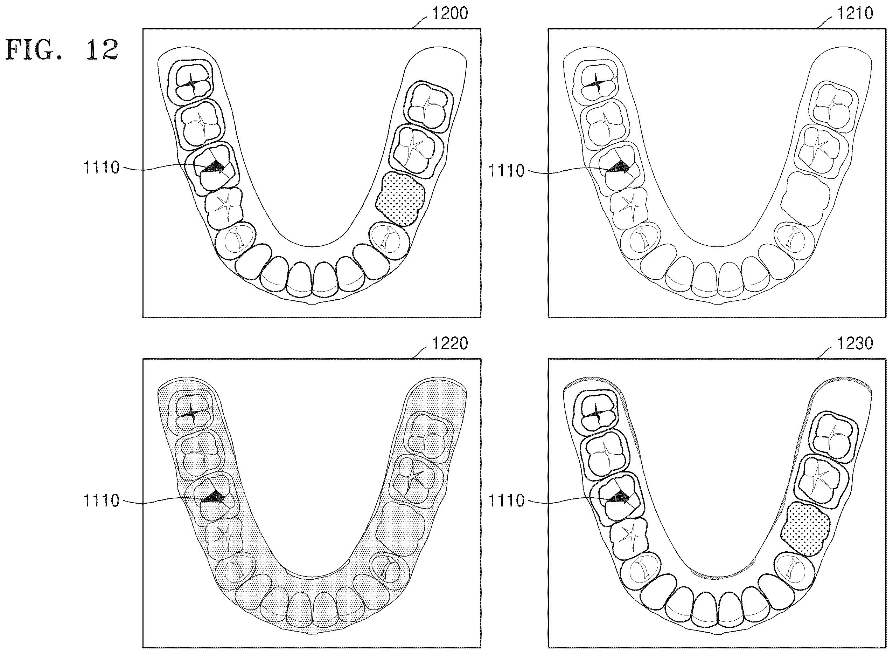

FIG. 12 is a view illustrating an operation of providing a three-dimensional oral image, according to an embodiment of the present disclosure.

Referring to FIGS. 1B, 2, and 12, a first interface 1200, a second interface 1210, a third interface 1220, and a fourth interface 1230 are illustrated in FIG. 12 according to an embodiment of the present disclosure.

In an embodiment of the present disclosure, the oral image processing device 100 may provide, through the display 200, interfaces including result images in a plurality of modes such that a user may acquire different pieces of information about the oral cavity. In this case, result images included in the first to fourth interfaces 1200, 1210, 1220, and 1230 may be three-dimensional oral images.

In an embodiment of the present disclosure, the plurality of modes may include a first mode that providing texture information on a plurality of oral elements included in the oral cavity, a second mode providing information other than the texture information, a third mode indicating reliability of three-dimensional oral images, and a fourth mode providing reliability and texture information on the three-dimensional oral images. In this case, the texture information may include color information on the three-dimensional oral image. The reliability of the three-dimensional oral images may be determined based on at least one of whether the number of two-dimensional oral images used to implement a three-dimensional oral image is greater than or equal to a reference number, the number of two-dimensional oral images captured in different poses for implementing a three-dimensional oral image of a specific region, and the density of a plurality of point clouds included in a three-dimensional oral image.

However, the present disclosure is not limited thereto, and the plurality of modes may further include other modes depending on the types of information that the oral image processing device 100 intends to provide to the user.

In an embodiment of the present disclosure, the first interface 1200 may be an interface including a result image provided in the first mode. The second interface 1210 may be an interface including a result image provided in the second mode. The third interface 1220 may be an interface including a result image in the third mode. The fourth interface 1230 may be an interface including a result image in the fourth mode.

In an embodiment of the present disclosure, the oral image processing device 100 may provide a dilated caries region 1110 by displaying the dilated caries region 1110 in the result image included in each of the first to fourth interfaces 1200, 1210, 1220, and 1230.

In this case, the oral image processing device 100 may display the dilated caries region 1110 in a preselected color to emphasize the dilated caries region 1110 for the user. In addition, the oral image processing device 100 may enhance visibility for the user by enlarging the size of the dilated caries region 1110 compared with the size in an oral image 110.

Through this, the oral image processing device 100 may provide information on a specific region (for example, a caries region) while providing various types of information about the oral cavity to the user.

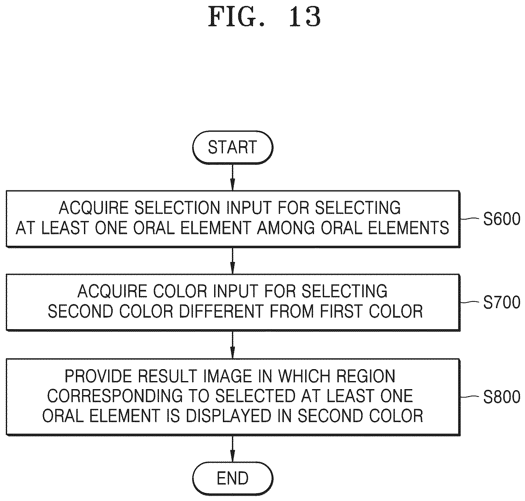

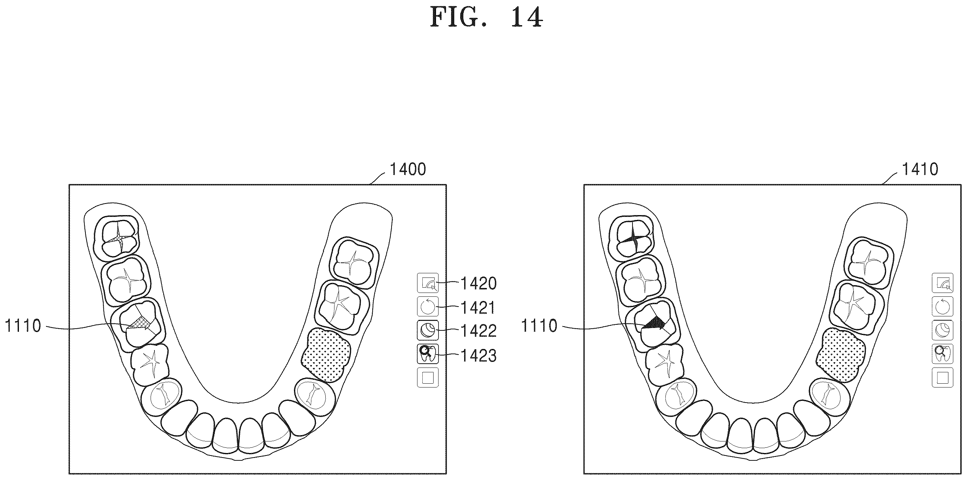

FIG. 13 is a flowchart illustrating an operation of displaying at least one oral element in a color different from an oral image, according to an embodiment of the present disclosure. FIG. 14 is a view illustrating an operation of displaying at least one oral element in a first color or a second color, according to an embodiment of the present disclosure.

Referring to FIGS. 1A, 2, and 13, in an embodiment of the present disclosure, the method of operating the oral image processing device 100 may include an operation S600 of acquiring a selection input for selecting at least one oral element among a plurality of oral elements included in an oral image 110.

According to an embodiment of the present disclosure, in the operation S600 of acquiring the selection input, the at least one processor 220 may acquire, through the input/output interface 230, a selection input for selecting at least one oral element among the plurality of oral elements. In an embodiment of the present disclosure, the at least one processor 220 may acquire, through the input/output interface 230, a selection input for selecting dental caries among the plurality of oral elements.

In an embodiment of the present disclosure, the method of operating the oral image processing device 100 may include an operation S700 of acquiring a color input for selecting a second color different from a first color of the at least one oral element selected in the oral image 110.

According to an embodiment of the present disclosure, in the operation S700 of acquiring the color input, the at least one processor 220 may acquire, through the input/output interface 230, a color input for selecting the second color different from the first color of the at least one oral element selected in the oral image 110. In an embodiment of the present disclosure, the at least one processor 220 may acquire, through the input/output interface 230, a color input for selecting the second color (for example, red) different from the first color of dental caries in the oral image 110.

In an embodiment of the present disclosure, the method of operating the oral image processing device 100 may include an operation S800 of providing, based on the selection input and the color input, a result image in which a region corresponding to the selected at least one oral element is displayed in the second color. In this case, the at least one oral element may be dental caries, and the region corresponding to the at least one oral element may be a dilated caries region.

According to an embodiment of the present disclosure, in the operation S800 of providing the result image, the at least one processor 220 may provide, through the display 200, the result image in which the region corresponding to the selected at least one oral element is displayed in the second color. In an embodiment of the present disclosure, the at least one processor 220 may provide, through the display 200, the result image in which the region corresponding to dental caries is displayed in the second color.

In an embodiment of the present disclosure, the result image displayed in operation S800 may include an oral image implemented in three dimensions.

However, the present disclosure is not limited thereto. The method of operating the oral image processing device 100 may not include the operation S700 of acquiring the color input for selecting the second color different from the first color. The second color may be a preset color different from the first color.

In this case, as the selection input for selecting at least one oral element among the plurality of oral elements included in the oral image 110 is acquired, the method of operating the oral image processing device 100 may provide, based on the selection input, a result image in which a region corresponding to the selected at least one oral element is displayed in the second color.

Referring to FIGS. 1B and 14, in an embodiment of the present disclosure, a fifth interface 1400 and a sixth interface 1410 are illustrated in FIG. 14.

In an embodiment of the present disclosure, a result image included in the fifth interface 1400 may display a selected oral element 1110 in the first color in the oral image 110. A result image included in the sixth interface 1410 may display the selected oral element 1110 in the second color different from the first color. In this case, the selected oral element 1110 may be dental caries.

In this case, when a color having a more prominent difference from the colors of other elements in the oral image 110 than the first color is selected, for example, red or a fluorescent color, the probability that a user finds the selected oral element such as dental caries may be greater in the sixth interface 1410 than in the fifth interface 1400.

In an embodiment of the present disclosure, the fifth interface 1400 or the sixth interface 1410 provided through the display 200 and including a result image may include a plurality of user interfaces for user convenience.

In an embodiment of the present disclosure, the plurality of user interfaces may include a first user interface 1421, a second user interface 1422, and a third user interface 1423.

In an embodiment of the present disclosure, the first user interface 1421 may be a user interface that receives an input for rotating the result image clockwise or counterclockwise. The oral image processing device 100 may rotate the result image included in the fifth interface 1400 or the sixth interface 1410 clockwise or counterclockwise according to the input acquired through the first user interface 1421.

In an embodiment of the present disclosure, the second user interface 1422 may be a user interface that receives an input for changing a mode of the result image. Referring to FIGS. 12 and 14, in an embodiment of the present disclosure, the oral image processing device 100 may provide the result image in any one of a plurality of modes that may include different pieces of information about the oral cavity. The oral image processing device 100 may change a mode in which the result image included in the fifth interface 1400 or the sixth interface 1410 is provided, according to the input acquired through the second user interface 1422.

In an embodiment of the present disclosure, when the oral image processing device 100 provides the result image in any one of first to fourth modes, the second user interface 1422 may acquire an input for sequential selection from the first mode to the fourth mode. The oral image processing device 100 may sequentially change the result image from the first mode to the fourth mode in response to the input acquired through the second user interface 1422.

In an embodiment of the present disclosure, the third user interface 1423 may be a user interface that receives a color selection input for selecting whether to display the selected oral element 1110 in the first color or the second color. The oral image processing device 100 may provide the selected oral element 1110 included in the result image in the first color or the second color according to the input acquired through the third user interface 1423.

However, the present disclosure is not limited thereto, and the fifth interface 1400 or the sixth interface 1410 may include other types of user interfaces for user convenience. In addition, arrangements, sizes, and shapes of the plurality of user interfaces may be changed.

In addition, the result images included in the fifth interface 1400 and the sixth interface 1410 may be three-dimensional oral images.

FIG. 15 is a view illustrating an operation of an oral image processing system according to an embodiment of the present disclosure. Hereinafter, the same components as those described with reference to FIGS. 1A, 1B, and 2, are denoted with the same reference numerals, and repeated descriptions thereof are omitted.

Referring to FIGS. 1A, 1B, 2, and 15, in an embodiment of the present disclosure, the oral image processing system may include a scanning device 1500 and an oral image processing device 100. The oral image processing device 100 and the scanning device 1500 may each include an input/output interface or a communication interface. The oral image processing device 100 and the scanning device 1500 may transmit and receive data through the input/output interfaces or communication interfaces.

In an embodiment of the present disclosure, the scanning device 1500 may be a device for scanning a target object, such as a medical device for acquiring images of the target object. In an embodiment of the present disclosure, the target object may include an object or a body part that is a scanning target of the scanning device 1500. In an embodiment of the present disclosure, the target object may include at least one of the oral cavity, artificial structures, and plaster models molded from the oral cavity or the artificial structures.

In an embodiment of the present disclosure, the scanning device 1500 may include an oral scanner configured to scan a patient's oral cavity. Hereinafter, for ease of description, the scanning device 1500 is described as an oral scanner 1500.

In an embodiment of the present disclosure, the oral scanner 1500 may be a handheld oral scanner configured to scan a patient's oral cavity while being held and moved by a user. The oral scanner 1500 may be inserted into the patient's oral cavity and may acquire images of a plurality of oral elements including at least one tooth by scanning the oral cavity in a non-contact manner.

In an embodiment of the present disclosure, the oral scanner 1500 may be of a type insertable into and withdrawable from the oral cavity. The oral scanner 1500 may scan the inside of a patient's oral cavity by capturing images using at least one image sensor (for example, an optical camera, an infrared camera, etc.).

In an embodiment of the present disclosure, the oral scanner 1500 may include a main body 1510 and a tip 1520. The main body 1510 may include a light projection unit that projects light and a light-receiving unit that receives light reflected from at least one oral element in the oral cavity. The main body 1510 may include a camera that photographs a target object (for example, at least one oral element) to acquire images.

The tip 1520 is a portion insertable into the oral cavity and may be detachably attached to the main body 1510. However, the present disclosure is not limited thereto, and the main body 1510 and the tip 1520 may be formed as an integrated structure.

In an embodiment of the present disclosure, the tip 1520 may include an optical path changing part to direct light projected from the main body 1510 toward a target object. In addition, the tip 1520 may direct light received from a target object (for example, at least one oral element) toward the main body 1510.

However, the present disclosure is not limited thereto. At least one of the light projection unit that projects light and the light-receiving unit that receives light reflected from at least one oral element in the oral cavity may be included in the tip 1520. In addition, the main body 1510 may include components such as a power storage unit for storing power required to operate the oral scanner 1500 is stored, and a processor for controlling the oral scanner 1500.

In an embodiment of the present disclosure, the oral scanner 1500 may acquire surface information on a target object as raw data by scanning the surface of at least one of teeth, gums, dental caries, artificial structures (for example, orthodontic appliances including brackets and wires, implants, artificial teeth, orthodontic auxiliary tools inserted into the oral cavity, or the like), and prostheses (for example, inlays, crowns, bridges, or the like).

In an embodiment of the present disclosure, the oral scanner 1500 may provide the acquired raw data to the oral image processing device 100 as an oral image 110. The oral image processing device 100 may render the acquired oral image 110 and generate and provide a result image that two-dimensionally or three-dimensionally representing at least one of teeth, gums, dental caries, artificial structures (for example, orthodontic appliances including brackets and wires, implants, artificial teeth, orthodontic auxiliary tools inserted into the oral cavity, or the like), and prostheses (for example, inlays, crowns, bridges, or the like).

According to an embodiment of the present disclosure, an oral image processing method may be implemented in the form of program instructions executable using various computer means and may be recorded on a computer-readable medium. In addition, an embodiment of the present disclosure may provide a computer-readable recording medium in which one or more programs including instructions for executing the oral image processing method is recorded.

The computer-readable recording medium may include any one of or a combination of program instructions, data files, data structures, and the like. Here, examples of the computer-readable recording medium may include: magnetic media such as hard disks, floppy disks, and magnetic tapes; optical recording media such as CD-ROMs and DVDs; magneto-optical media such as floptical disks; and hardware such as ROMs, RAMs, and flash memories specifically configured to store program instructions and execute the program instructions.

Herein, the computer-readable recording medium may be provided in the form of a non-transitory storage medium. Herein, the term "non-transitory storage medium" may refer to a tangible storage device. In addition, the "non-transitory storage medium" may include a buffer in which data is temporarily stored.

According to various embodiments of the present disclosure, the oral image processing method may be included and provided in a computer program product. The computer program product may be distributed in the form of a machine-readable storage medium (for example, compact disc read only memory (CD-ROM)). In addition, the computer program product may be distributed (for example, downloaded or uploaded) online via an application store (for example, PlayStore^{™}), or between two user devices (for example, smartphones) directly. Specifically, according to an embodiment of the present disclosure, the computer program product may include a storage medium on which a program including at least one instruction for performing the oral image processing method is recorded.

Although the embodiments have been described in detail above, the scope of the present disclosure is not limited thereto, and various modifications and improvements by those skilled in the art using the basic concept of the present disclosure defined in the following claims also fall within the scope of the present disclosure.

## Claims

1. A method of operating an oral image processing device, the method comprising:
acquiring an oral image comprising a plurality of oral elements;
based on the oral image, acquiring a segmentation map in which a plurality of oral element regions respectively corresponding to the plurality of oral elements are defined by segmentation;
identifying a caries region corresponding to dental caries among the plurality of oral element regions in the segmentation map;
dilating the caries region to acquire an adjusted segmentation map in which the caries region among the plurality of oral element regions is dilated; and
based on the adjusted segmentation map, providing a result image in which the dental caries among the plurality of oral elements is displayed as the dilated caries region larger than the identified caries region.

2. The method of claim 1, wherein the providing of the result image comprises:
based on the oral image and the adjusted segmentation map, generating a three-dimensional oral image in which the dental caries among the plurality of oral elements is displayed as the dilated caries region; and
providing the three-dimensional oral image as the result image.

3. The method of claim 2, further comprising:
extracting, from the segmentation map, a caries masking map comprising information on the caries region corresponding to the dental caries among the plurality of oral elements; and
generating an adjusted caries masking map by dilating the caries region included in the caries masking map,
wherein the acquiring of the adjusted segmentation map comprises acquiring, based on the adjusted caries masking map and the segmentation map, the adjusted segmentation map in which the caries region is dilated.

4. The method of claim 3, wherein, among the plurality of oral regions included in the adjusted segmentation map, the dilated caries region has a first weight, and remaining regions have a second weight, and
the first weight is greater than the second weight.

5. The method of claim 4, wherein the oral image comprises a plurality of oral images,
the adjusted segmentation map comprises a plurality of adjusted segmentation maps respectively corresponding to the plurality of oral images, and
in each of the plurality of adjusted segmentation maps, the dilated caries region has the first weight, and the remaining regions have the second weight,
wherein, in the generating of the three-dimensional oral image, all of the plurality of adjusted segmentation maps are integrated to generate the three-dimensional oral image such that a region, among the plurality of oral regions, having the first weight greater than the second weight is displayed as the dilated caries region.

6. The method of claim 5, wherein the generating of the three-dimensional oral image comprises:
generating a plurality of point clouds based on the plurality of oral images; and
based on the plurality of point clouds and the plurality of adjusted segmentation maps, generating the three-dimensional oral image comprising a plurality of voxels comprising the plurality of point clouds.

7. The method of claim 6, further comprising:
generating a plurality of meshes comprising information on the three-dimensional oral image by using the plurality of voxels; and
generating a post-processed three-dimensional oral image by using the plurality of meshes.

8. The method of claim 1, wherein the acquiring of the segmentation map comprises providing the oral image as an input to a segmentation map extraction module and acquiring the segmentation map as an inference result of the segmentation map extraction module, and
the segmentation map extraction module comprises an artificial intelligence model pretrained using data sets, each of the data sets comprising a training oral image comprising a plurality of oral elements and labeling information in which the plurality of oral elements included in the training oral images are labeled.

9. The method of claim 1, wherein, in the acquiring of the segmentation map, tooth regions corresponding to teeth and the caries region corresponding to the dental caries are defined by selective segmentation based on the acquired oral image to acquire the segmentation map.

10. The method of claim 1, wherein the providing of the result image comprises:
acquiring a selection input for selecting at least one oral element among the plurality of oral elements; and
based on the selection input, providing the result image such that a region corresponding to the selected at least one oral element is displayed in a second color different from a first color representing the oral image.

11. An oral image processing device comprising:
a memory storing at least one instruction; and
at least one processor executing the at least one instruction stored in the memory,
wherein the at least one processor executes the at least one instruction to:
acquire an oral image comprising a plurality of oral elements;
acquire, based on the oral image, a segmentation map in which a plurality of oral element regions respectively corresponding to the plurality of oral elements are defined by segmentation;
identify a caries region corresponding to dental caries among the plurality of oral element regions in the segmentation map;
dilate the caries region to acquire an adjusted segmentation map in which the caries region among the plurality of oral element regions is dilated ; and
provide, based on the adjusted segmentation map, a result image in which the dental caries among the plurality of oral elements is displayed as the dilated caries region larger than the identified caries region.

12. The oral image processing device of claim 11, wherein the at least one processor executes the at least one instruction to:
generate, based on the oral image and the adjusted segmentation map, a three-dimensional oral image in which the dental caries among the plurality of oral elements is displayed as the dilated caries region; and
provide the three-dimensional oral image as the result image.

13. The oral image processing device of claim 12, wherein the at least one processor executes the at least one instruction to:
extract, from the segmentation map, a caries masking map comprising information on the caries region corresponding to the dental caries among the plurality of oral elements;
generate an adjusted caries masking map by dilating the caries region included in the caries masking map; and
acquire, based on the adjusted caries masking map and the segmentation map, the adjusted segmentation map in which the caries region is dilated.

14. The oral image processing device of claim 13, wherein the at least one processor executes the at least one instruction such that:
among the plurality of oral regions included in the adjusted segmentation map, the dilated caries region has a first weight, and remaining regions have a second weight; and
the first weight is greater than the second weight.

15. The oral image processing device of claim 14, wherein the oral image comprises a plurality of oral images,
the adjusted segmentation map comprises a plurality of adjusted segmentation maps respectively corresponding to the plurality of oral images, and
in each of the plurality of adjusted segmentation maps, the dilated caries region has the first weight, and the remaining regions have the second weight,
wherein the at least one processor executes the at least one instruction to generate the three-dimensional oral image by integrating all of the plurality of adjusted segmentation maps such that a region, among the plurality of oral regions, having the first weight greater than the second weight is displayed as the dilated caries region.

16. The oral image processing device of claim 15, wherein the at least one processor executes the at least one instruction to:
generate a plurality of point clouds based on the plurality of oral images; and
generate, based on the plurality of point clouds and the plurality of adjusted segmentation maps, the three-dimensional oral image comprising a plurality of voxels comprising the plurality of point clouds.

17. The oral image processing device of claim 16, wherein the at least one processor executes the at least one instruction to:
generate a plurality of meshes comprising information on the three-dimensional oral image by using the plurality of voxels; and
generate a post-processed three-dimensional oral image by using the plurality of meshes.

18. The oral image processing device of claim 11, wherein the at least one processor executes the at least one instruction to provide the oral image as an input to a segmentation map extraction module and acquire the segmentation map as an inference result of the segmentation map extraction module, and
the segmentation map extraction module comprises an artificial intelligence model pretrained using data sets, each of the data sets comprising a training oral image comprising a plurality of oral elements and labeling information in which the plurality of oral elements included in the training oral images are labeled.

19. The oral image processing device of claim 11, wherein the at least one processor executes the at least one instruction to:
acquire a selection input for selecting at least one oral element among the plurality of oral elements; and
provide, based on the selection input, the result image such that a region corresponding to the selected at least one oral element is displayed in a second color different from a first color representing the oral image.

20. A non-transitory computer-readable recording medium storing at least one instruction executable by at least one processor of an oral image processing device,
wherein the at least one processor of the oral image processing device executes the at least one instruction to cause the oral image processing device to:
acquire an oral image comprising a plurality of oral elements;
acquire, based on the oral image, a segmentation map in which a plurality of oral element regions respectively corresponding to the plurality of oral elements are defined by segmentation;
identify a caries region corresponding to dental caries among the plurality of oral element regions in the segmentation map;
dilate the caries region to acquire an adjusted segmentation map in which the caries region among the plurality of oral element regions is dilated; and
provide, based on the adjusted segmentation map, a result image in which the dental caries among the plurality of oral elements is displayed as the dilated caries region larger than the identified caries region.
